# EUROPEAN PATENT APPLICATION

(11) **EP 4 487 867 A1**
(43) Date of publication of application: **08.01.2025**
(21) Application number: 23762951.4
(22) Date of filing: 02.03.2023
(51) Int. Cl.: A61K 38/20, C07K 14/55, C07K 19/00, A61K 47/68, A61P 35/00, A61P 37/02

(54) **PHARMACEUTICAL COMPOSITION OF IL2 MUTANT-ANTIBODY FC BLOCK FUSION PROTEIN AND USE THEREOF**

(30) Priority: 03.03.2022 CN 202210210240; 28.02.2023 CN 202310181936
(71) Applicant: Hainan Simcere Pharmaceutical Co., Ltd., Haikou, Hainan 570311 (CN)
(72) Inventor: LI, Shengnan, Shanghai 201318 (CN); WANG, Shuai, Shanghai 201318 (CN); LI, Xinxin, Nanjing, Jiangsu 210042 (CN); YAN, Yuxi, Shanghai 201318 (CN); HU, Jianzhong, Nanjing, Jiangsu 210042 (CN); TANG, Renhong, Shanghai 201318 (CN); REN, Jinsheng, Nanjing, Jiangsu 210042 (CN)
(74) Representative: KATZAROV S.A.
(86) International application number: PCT/CN2023/079252
(87) International publication number: WO 2023/165553

(57) **Abstract**

Provided are a pharmaceutical composition of an IL2 mutant-antibody Fc block fusion protein and a preparation method therefor, a lyophilized preparation and a preparation or reconstitution method therefor, an obtained reconstitution solution, a corresponding product, a treatment method for an autoimmune disease and a proliferative disease, and corresponding pharmaceutical use. The pharmaceutical composition comprises: a fusion protein containing an IL2 mutant and an antibody Fc block, a buffer, an osmotic pressure adjuster, and a surfactant, and has good stability.

## Description

### TECHNICAL FIELD

The present invention relates to the field of pharmaceutical formulations, in particular to a pharmaceutical composition of IL2 mutant-antibody Fc block fusion protein and use thereof.

### BACKGROUND

Interleukin-2 (IL2) was originally identified as T-cell growth factor (TCGF). It has been found that IL-2 can bind to its receptor and activate the proliferation and activation of immune cells such as T cells and NK cells. IL-2 receptors include IL-2Rα subunit (CD25), IL-2Rβ subunit (CD122), and IL-2Rγ subunit (CD132). Different subunits can form receptor complexes with different affinities, including high-affinity receptor IL-2Rαβγ, intermediate-affinity receptor IL-2Rβγ, and low-affinity receptor IL-2Rα or IL-2Rαβ. Different cells express different types of IL-2R subunits. For example, under resting conditions, traditional T cells, CD4⁺ T and CD8⁺ T, typically express IL-2 receptor β (IL-2Rβ, CD122) and IL-2 receptor γ (IL-2Rγ, CD132), and hardly express IL-2 receptor α (IL-2Rα, CD25), while regulatory T cells (Tregs) constitutively and highly express IL-2Rα in addition to IL-2Rβ and IL-2Rγ.

At present, researchers adopt IL2 or mutants thereof to activate immune cells or a subset of immune cells, thereby treating tumors or autoimmune diseases. For example, high doses of IL2 have been approved for the treatment of malignant melanoma or metastatic renal cell carcinoma, and an IL2 polyethylene glycol conjugate drug, NKTR-358, has been approved for clinical trials of autoimmune diseases. Further development of a new IL2 mutant to improve its stability and yield and/or change its binding ability to a certain type of receptor complexes is of great significance for developing IL-2 drugs. Meanwhile, IL2 is a macromolecular protein, which is prone to stability problems such as particle formation and aggregation in solution. Therefore, after obtaining a novel IL2 mutant, it is of great significance to develop a formulation system suitable for the novel IL2 mutant and to ensure the stability of the novel IL2 mutant in solution.

### SUMMARY

The present invention provides a pharmaceutical composition and a preparation method therefor, a lyophilized formulation and a preparation or reconstitution method therefor, a reconstituted solution obtained according to the reconstitution method, corresponding pharmaceutical use, a method for treating an autoimmune disease, and an article of manufacture.

In a first aspect, the present invention provides a pharmaceutical composition, which comprises a fusion protein comprising an IL2 mutant and an antibody Fc block, a buffer, an osmotic pressure regulator, and a surfactant;
preferably, the IL2 mutant comprises at least Y31V, A73L, and H79Q mutations compared to wild-type IL2;
preferably, the IL2 mutant further comprises one or more of a V91R mutation, an H16E mutation and, a D20A mutation, for example, an H16E mutation and a V91R mutation;
more preferably, the IL2 mutant has an amino acid sequence having at least 80% identity, e.g., at least 80%, 85%, 90%, 91%, 92%, 93%, 94%, 95%, 96%, 97%, 98%, 99%, or 100% identity to the sequence set forth in any one of SEQ ID NOs: 3 and 8-11.

The amino acid sequence of the wild-type IL2 is set forth in SEQ ID NO: 2.

In some embodiments, the fusion protein sequentially comprises, from the N-terminus to the C-terminus:
(1) the IL2 mutant, a linker, and the antibody Fc block, or
(2) the antibody Fc block, a linker, and the IL2 mutant;
   preferably, the fusion protein forms a homodimer by dimerization of the antibody Fc block; preferably, the linker has an amino acid sequence of (G₄S)ₙ, wherein n is selected from 1, 2, 3, 4, 5, or 6;
   preferably, the linker has an amino acid sequence having at least 80% identity, e.g., at least 80%, 85%, 90%, 91%, 92%, 93%, 94%, 95%, 96%, 97%, 98%, 99%, or 100% identity to the sequence set forth in SEQ ID NO 12;
   preferably, the antibody Fc block comprises an N297G mutation;
   preferably, the antibody Fc block has an amino acid sequence having at least 80% identity, e.g., at least 80%, 85%, 90%, 91%, 92%, 93%, 94%, 95%, 96%, 97%, 98%, 99%, or 100% identity to the sequence set forth in SEQ ID NO 13;
   more preferably, the fusion protein has an amino acid sequence having at least 80% identity, e.g., at least 80%, 85%, 90%, 91%, 92%, 93%, 94%, 95%, 96%, 97%, 98%, 99%, or 100% identity to the sequence set forth in SEQ ID NOs: 14-18.

In some embodiments, the wild-type IL2 has an amino acid sequence set forth in SEQ ID NO: 1 or 2.

In some embodiments, the concentration of the fusion protein is about 1-50 mg/mL;
for example, the concentration of the fusion protein is about 1-40 mg/mL, 1-30 mg/mL, 1-20 mg/mL, 1-15 mg/mL, 1-10 mg/mL, 5-50 mg/mL, 5-40 mg/mL, 5-30 mg/mL, 5-20 mg/mL, 5-19 mg/mL, 5-18 mg/mL, 5-17 mg/mL, 5-16 mg/mL, 5-15 mg/mL, 5-14 mg/mL, 5-13 mg/mL, 5-12 mg/mL, 5-11 mg/mL, 5-10 mg/mL, 5-9 mg/mL, 5-8 mg/mL, 5-6 mg/mL, 10-50 mg/mL, 10-40 mg/mL, 10-30 mg/mL, 10-20 mg/mL, 10-15 mg/mL, 20-50 mg/mL, 20-40 mg/mL, 20-30 mg/mL, 30-50 mg/mL, 30-40 mg/mL, or 40-50 mg/mL;
for example, the concentration of the fusion protein is about 1 mg/mL, 2 mg/mL, 3 mg/mL, 4 mg/mL, 5 mg/mL, 6 mg/mL, 7 mg/mL, 8 mg/mL, 9 mg/mL, 10 mg/mL, 11 mg/mL, 12 mg/mL, 13 mg/mL, 14 mg/mL, 15 mg/mL, 16 mg/mL, 17 mg/mL, 18 mg/mL, 19 mg/mL, 20 mg/mL, 21 mg/mL, 22 mg/mL, 23 mg/mL, 24 mg/mL, 25 mg/mL, 26 mg/mL, 27 mg/mL, 28 mg/mL, 29 mg/mL, 30 mg/mL, 31 mg/mL, 32 mg/mL, 33 mg/mL, 34 mg/mL, 35 mg/mL, 36 mg/mL, 37 mg/mL, 38 mg/mL, 39 mg/mL, 40 mg/mL, 41 mg/mL, 42 mg/mL, 43 mg/mL, 44 mg/mL, 45 mg/mL, 46 mg/mL, 47 mg/mL, 48 mg/mL, 49 mg/mL, or 50 mg/mL; or a concentration range value composed of the concentration values described above.

Preferably, the concentration of the fusion protein is about 2 mg/mL or 5 mg/mL.

In some embodiments, the buffer is selected from an acetic acid-sodium acetate buffer, a citric acid-sodium citrate buffer, a histidine-histidine hydrochloride buffer, a succinic acid-sodium succinate buffer, or a disodium hydrogen phosphate-sodium dihydrogen phosphate buffer, preferably an acetic acid-sodium acetate buffer; and/or
the concentration of the buffer is about 1-100 mM;
for example, the concentration of the buffer is about 1-90 mM, 1-80 mM, 1-70 mM, 1-60 mM, 1-50 mM, 1-40 mM, 1-30 mM, 1-20 mM, 1-10 mM, 10-90 mM, 10-80 mM, 10-70 mM, 10-60 mM, 10-50 mM, 10-40 mM, 10-30 mM, 10-20 mM, 20-90 mM, 20-80 mM, 20-70 mM, 20-60 mM, 20-50 mM, 20-40 mM, 20-30 mM, 30-90 mM, 30-80 mM, 30-70 mM, 30-60 mM, 30-50 mM, 30-40 mM, 40-90 mM, 40-80 mM, 40-70 mM, 40-60 mM, 40-50 mM, 50-90 mM, 50-80 mM, 50-70 mM, 50-60 mM, 60-90 mM, 60-80 mM, 60-70 mM, 70-90 mM, or 80-90 mM;
for example, the concentration of the buffer is about 1 mM, 5 mM, 10 mM, 15 mM, 20 mM, 25 mM, 30 mM, 35 mM, 40 mM, 45 mM, 50 mM, 55 mM, 60 mM, 65 mM, 70 mM, 75 mM, 80 mM, 85 mM, 90 mM, 95 mM, or 100 mM; or a concentration range value composed of the concentration values described above;
preferably, the concentration of the buffer is about 20 mM; and/or
the buffer has a pH value of about 4.5-8.0;
for example, the buffer has a pH value of about 4.5-7.5, 4.5-7.0, 4.5-6.5, 4.5-6.0, 4.5-5.5, 4.5-5.0, 5.0-8.0, 5.0-7.5, 5.0-7.0, 5.0-6.5, 5.0-6.0, 5.0-5.5, 5.5-8.0, 6-7.5, 6.5-7.5, 7.0-7.5, or 7.5-8.0;
for example, the buffer has a pH value of about 4.5, 5.0, 5.5, 6.0, 6.5, 7.0, 7.5, or 8.0, or a pH range value composed of the pH values described above;
preferably, the buffer has a pH value of about 5.5.

In some embodiments, the osmotic pressure regulator is selected from a salt, an amino acid, a sugar or sugar alcohol, or a combination thereof; preferably, the salt is selected from sodium chloride, potassium chloride, or arginine hydrochloride; preferably, the amino acid is selected from glycine, arginine, histidine, glutamic acid, or methionine; preferably, the sugar or sugar alcohol is selected from sucrose, trehalose, mannitol, or sorbitol;
preferably, the osmotic pressure regulator is selected from sodium chloride, glycine, arginine hydrochloride, sucrose, trehalose, mannitol, or sorbitol, more preferably sucrose or arginine hydrochloride;
more preferably, the concentration of the sugar or sugar alcohol is about 1-15% w/v, and the concentration of the salt is about 50-200 mM; for example, the concentration of the sugar or sugar alcohol is about 1-10% w/v, 1-5% w/v, or 5-10% w/v; for example, the concentration of the sugar or sugar alcohol is about 1% w/v, 1.5% w/v, 2% w/v, 2.5% w/v, 3% w/v, 3.5% w/v, 4% w/v, 4.5% w/v, 5% w/v, 5.5% w/v, 6% w/v, 6.5% w/v, 7% w/v, 7.5% w/v, 8% w/v, 8.5% w/v, 9% w/v, 9.5% w/v, 10% w/v, 10.5% w/v, 11% w/v, 11.5% w/v, 12% w/v, 12.5% w/v, 13% w/v, 13.5% w/v, 14% w/v, 14.5% w/v, 15% w/v, or a concentration range value composed of the concentration values described above, preferably 4.5% w/v or 8% w/v.

More preferably, the concentration of the amino acid is about 1-15% w/v, for example, the concentration of the amino acid is about 1-10% w/v, 1-5% w/v, or 5-10% w/v; for example, the concentration of the amino acid is about 1% w/v, 1.5% w/v, 2% w/v, 2.5% w/v, 3% w/v, 3.5% w/v, 4% w/v, 4.5% w/v, 5% w/v, 5.5% w/v, 6% w/v, 6.5% w/v, 7% w/v, 7.5% w/v, 8% w/v, 8.5% w/v, 9% w/v, 9.5% w/v, 10% w/v, 10.5% w/v, 11% w/v, 11.5% w/v, 12% w/v, 12.5% w/v, 13% w/v, 13.5% w/v, 14% w/v, 14.5% w/v, 15% w/v, or a concentration range value composed of the concentration values described above, preferably 2% w/v.

More preferably, the concentration of the salt is about 50-200 mM; for example, the concentration of the salt is about 50-150 mM, 50-100 mM, or 100-150 mM; for example, the concentration of the salt is about 50 mM, 60 mM, 70 mM, 80 mM, 90 mM, 100 mM, 110 mM, 120 mM, 130 mM, 140 mM, 150 mM, 160 mM, 170 mM, 180 mM, 190 mM, or 200 mM, or a concentration range value composed of the concentration values described above, preferably 140 mM.

More preferably, the osmotic pressure regulator is sucrose or arginine hydrochloride.

In some embodiments, the surfactant is polysorbate-80 (PS-80), polysorbate 20 (PS-20), or poloxamer; and/or
the concentration of the surfactant is about 0.01-0.1% w/v, such as 0.01% w/v-0.05% w/v, 0.05% w/v-0.1% w/v, or 0.02% w/v-0.08% w/v, e.g., about 0.01% w/v, 0.02% w/v, 0.03% w/v, 0.04% w/v, 0.05% w/v, 0.06% w/v, 0.07% w/v, 0.08% w/v, 0.09% w/v, or 1% w/v, or a concentration range value composed of the concentration values described above, preferably about 0.04% w/v.

In some embodiments, the pharmaceutical composition comprises the fusion protein, the acetic acid-sodium acetate buffer, sucrose, and polysorbate-80; preferably, the pharmaceutical composition comprises about 1-50 mg/mL fusion protein, about 1-100 mM acetic acid-sodium acetate buffer, about 1-15% w/v sucrose, and about 0.01-0.1% w/v polysorbate-80, with a pH value of about 4.5-6.0;
more preferably, the pharmaceutical composition comprises about 1-30 mg/mL, 5-30 mg/mL, 10-30 mg/mL, 10-25 mg/mL, 10-20 mg/mL, 10-15 mg/mL, 15-20 mg/mL, 15-30 mg/mL, or 20-30 mg/mL fusion protein, about 10-50 mM or 10-30 mM acetic acid-sodium acetate buffer, about 1-15% w/v, 1-10% w/v, 5-15% w/v, 5-10% w/v, or 10-15% w/v sucrose, and about 0.01-0.1% w/v or 0.02-0.06% w/v polysorbate-80, with a pH value of about 4.5-6.0 or 4.5-5.5;
more preferably, the pharmaceutical composition comprises about 1-10 mg/mL fusion protein, about 10-30 mM acetic acid-sodium acetate buffer, about 5-10% w/v sucrose, and about 0.01-0.1% w/v polysorbate-80, with a pH value of about 4.5-6.0;
more preferably, the pharmaceutical composition comprises about 2 mg/mL or 5 mg/mL fusion protein, about 20 mM acetic acid-sodium acetate buffer, about 8% w/v sucrose, and about 0.04% w/v polysorbate-80, with a pH value of about 5.5.

In some embodiments, the pharmaceutical composition comprises the fusion protein, the acetic acid-sodium acetate buffer, arginine hydrochloride, and polysorbate-80; preferably, the pharmaceutical composition comprises about 1-50 mg/mL fusion protein, about 1-100 mM acetic acid-sodium acetate buffer, about 50-200 mM arginine hydrochloride, and about 0.01-0.1% w/v polysorbate-80, with a pH value of about 4.5-6.0;
more preferably, the pharmaceutical composition comprises about 1-30 mg/mL, 5-30 mg/mL, 10-30 mg/mL, 10-25 mg/mL, 10-20 mg/mL, 10-15 mg/mL, 15-20 mg/mL, 15-30 mg/mL, or 20-30 mg/mL fusion protein, about 10-50 mM or 10-30 mM acetic acid-sodium acetate buffer, about 50-200 mM, 50-150 mM, or 100-150 mM arginine hydrochloride, and about 0.01-0.1% w/v or 0.02-0.06% w/v polysorbate-80, with a pH value of about 4.5-6.0 or 4.5-6.0;
more preferably, the pharmaceutical composition comprises about 1-10 mg/mL fusion protein, about 10-30 mM acetic acid-sodium acetate buffer, about 120-150 mM arginine hydrochloride, and about 0.01-0.1% w/v polysorbate-80, with a pH value of about 4.5-6.0;
more preferably, the pharmaceutical composition comprises about 2 mg/mL or 5 mg/mL fusion protein, about 20 mM acetic acid-sodium acetate buffer, about 140 mM arginine hydrochloride, and about 0.04% w/v or 0.06% w/v polysorbate-80, with a pH value of about 5.5.

In some embodiments, the pharmaceutical composition is an intravenous injection, an intramuscular injection, or a subcutaneous injection, preferably a subcutaneous injection.

In a second aspect, the present invention further provides a method for preparing the aforementioned pharmaceutical composition. The method comprises a step of mixing the fusion protein with the buffer, the osmotic pressure regulator, and the surfactant; preferably, the method comprises a step of exchanging a stock solution of the fusion protein into the buffer by ultrafiltration concentration.

In a third aspect, the present invention further provides a lyophilized formulation, wherein the lyophilized formulation is formed by lyophilizing the aforementioned pharmaceutical composition.

In a fourth aspect, the present invention further provides a method for preparing the aforementioned lyophilized formulation, wherein the method comprises a step of lyophilizing the aforementioned pharmaceutical composition.

In a fifth aspect, the present invention further provides a method for preparing a reconstituted solution of an antibody Fc block fusion protein comprising an IL2 mutant, wherein the method comprises reconstituting the aforementioned lyophilized formulation with a solvent; preferably, the solvent is water for injection.

In a sixth aspect, the present invention further provides a reconstituted solution of the antibody Fc block fusion protein comprising the IL2 mutant prepared according to the aforementioned method.

In a seventh aspect, the present invention further provides use of the aforementioned pharmaceutical composition, lyophilized formulation, or reconstituted solution in the manufacture of a medicament for treating an autoimmune disease or a proliferative disease;
preferably, the autoimmune disease is selected from rheumatoid arthritis, ankylosing spondylitis, systemic lupus erythematosus, cutaneous lupus erythematosus, lupus nephritis, IgA nephropathy, Sjogren's syndrome, polymyositis, dermatomyositis, scleroderma, psoriasis, plaque psoriasis, alopecia areata, multiple sclerosis, amyotrophic lateral sclerosis, inflammatory bowel disease, ulcerative colitis, Crohn's disease, graft-versus-host disease, organ transplant rejection, autoimmune hepatitis, type I diabetes, autoimmune vasculitis, eczema, or asthma;
preferably, the proliferative disease is selected from a neoplasm, a solid tumor, a hematologic tumor, malignant ascites, or malignant pleural effusion; wherein the solid tumor can be benign or malignant, primary or metastatic, and the malignant solid tumor can be a cancer or a sarcoma, such as epithelial cell carcinoma, endothelial cell carcinoma, squamous cell carcinoma, teratoma, lung tumor, papillomavirus-induced cancer, adenocarcinoma, carcinoma, melanoma, angiosarcoma, neuroblastoma, metastatic lung cancer, non-small cell lung cancer, small cell lung cancer, breast cancer, Merkel cell carcinoma, ovarian cancer, renal cell carcinoma, metastatic renal cancer, head and neck cancer, bladder cancer, or non-muscle invasive bladder cancer; the hematologic tumor can be selected from leukemia, lymphoma, and multiple myeloma, such as B-cell lymphoma, T-cell lymphoma, cutaneous T-cell lymphoma, and T-cell large granular lymphocytic leukemia.

In an eighth aspect, the present invention further provides the aforementioned pharmaceutical composition, lyophilized formulation, or reconstituted solution for use in treating an autoimmune disease or a proliferative disease;
preferably, the autoimmune disease is selected from rheumatoid arthritis, ankylosing spondylitis, systemic lupus erythematosus, cutaneous lupus erythematosus, lupus nephritis, IgA nephropathy, Sjogren's syndrome, polymyositis, dermatomyositis, scleroderma, psoriasis, plaque psoriasis, alopecia areata, multiple sclerosis, amyotrophic lateral sclerosis, inflammatory bowel disease, ulcerative colitis, Crohn's disease, graft-versus-host disease, organ transplant rejection, autoimmune hepatitis, type I diabetes, autoimmune vasculitis, eczema, or asthma;
preferably, the proliferative disease is selected from a neoplasm, a solid tumor, a hematologic tumor, malignant ascites, or malignant pleural effusion; wherein the solid tumor can be benign or malignant, primary or metastatic, and the malignant solid tumor can be a cancer or a sarcoma, such as epithelial cell carcinoma, endothelial cell carcinoma, squamous cell carcinoma, teratoma, lung tumor, papillomavirus-induced cancer, adenocarcinoma, carcinoma, melanoma, angiosarcoma, neuroblastoma, metastatic lung cancer, non-small cell lung cancer, small cell lung cancer, breast cancer, Merkel cell carcinoma, ovarian cancer, renal cell carcinoma, metastatic renal cancer, head and neck cancer, bladder cancer, or non-muscle invasive bladder cancer; the hematologic tumor can be selected from leukemia, lymphoma, and multiple myeloma, such as B-cell lymphoma, T-cell lymphoma, cutaneous T-cell lymphoma, and T-cell large granular lymphocytic leukemia.

In a ninth aspect, the present invention further provides a method for treating an autoimmune disease, wherein the method comprises administering to a subject an effective amount of the aforementioned pharmaceutical composition or reconstituted solution; preferably, the autoimmune disease is selected from rheumatoid arthritis, ankylosing spondylitis, systemic lupus erythematosus, cutaneous lupus erythematosus, lupus nephritis, IgA nephropathy, Sjogren's syndrome, polymyositis, dermatomyositis, scleroderma, psoriasis, plaque psoriasis, alopecia areata, multiple sclerosis, amyotrophic lateral sclerosis, inflammatory bowel disease, ulcerative colitis, Crohn's disease, graft-versus-host disease, organ transplant rejection, autoimmune hepatitis, type I diabetes, autoimmune vasculitis, eczema, or asthma.

In some embodiments, the effective amount is 0.001-10 mpk, such as 0.001 mpk, 0.002 mpk, 0.003 mpk, 0.004 mpk, 0.005 mpk, 0.006 mpk, 0.007 mpk, 0.008 mpk, 0.009 mpk, 0.01 mpk, 0.02 mpk, 0.03 mpk, 0.04 mpk, 0.05 mpk, 0.06 mpk, 0.07 mpk, 0.08 mpk, 0.09 mpk, 0.1 mpk, 0.2 mpk, 0.3 mpk, 0.4 mpk, 0.5 mpk, 0.6 mpk, 0.7 mpk, 0.8 mpk, 0.9 mpk, 1 mpk, 2 mpk, 3 mpk, 4 mpk, 5 mpk, 6 mpk, 7 mpk, 8 mpk, 9 mpk, or 10 mpk, or an effective amount range value composed of the effective amounts described above.

In a tenth aspect, the present invention further provides a method for treating a proliferative disease, wherein the method comprises administering to a subject an effective amount of the aforementioned pharmaceutical composition or reconstituted solution; preferably, the proliferative disease is selected from a neoplasm, a solid tumor, a hematologic tumor, malignant ascites, or malignant pleural effusion; wherein the solid tumor can be benign or malignant, primary or metastatic, and the malignant solid tumor can be a cancer or a sarcoma, such as epithelial cell carcinoma, endothelial cell carcinoma, squamous cell carcinoma, teratoma, lung tumor, papillomavirus-induced cancer, adenocarcinoma, carcinoma, melanoma, angiosarcoma, neuroblastoma, metastatic lung cancer, non-small cell lung cancer, small cell lung cancer, breast cancer, Merkel cell carcinoma, ovarian cancer, renal cell carcinoma, metastatic renal cancer, head and neck cancer, bladder cancer, or non-muscle invasive bladder cancer; the hematologic tumor can be selected from leukemia, lymphoma, and multiple myeloma, such as B-cell lymphoma, T-cell lymphoma, cutaneous T-cell lymphoma, and T-cell large granular lymphocytic leukemia.

In an eleventh aspect, the present invention further provides an article of manufacture, which comprises a container containing the aforementioned pharmaceutical composition, lyophilized formulation, or reconstituted solution.

### TERMINOLOGY AND DEFINITIONS

Unless otherwise defined herein, scientific and technical terms used in correlation with the present invention shall have the meanings that are commonly understood by those skilled in the art.

Unless otherwise indicated, the term "IL2" or "IL-2" described herein refers to any natural or recombinant IL-2 from any vertebrate source, including mammals such as primates (e.g., humans) and rodents (e.g., mice and rats), and domestic or agricultural mammals. The "IL2" or "IL-2" described herein includes unprocessed IL-2 (e.g., IL-2 comprising an N-terminal signal peptide) and any form of processed IL-2 produced from cells. The "IL2" or "IL-2" described herein also includes natural variants and fragments of IL-2, such as splice variants or allelic variants. The "IL2" or "IL-2" described herein also includes non-naturally occurring mutants, such as IL-2 mutants artificially modified by genetic engineering.

"Wild-type IL-2" is a form of IL-2 that maintains a wild-type amino acid at each mutated amino acid position of an IL-2 mutant but otherwise is identical to the IL-2 mutant. Illustratively, if the IL-2 mutant is unprocessed IL-2, the wild-type form of the mutant is unprocessed IL-2; if the IL-2 mutant is processed IL-2, the wild-type form of the mutant is processed IL-2; if the IL-2 mutant is a truncated form of IL-2, the wild-type form of the mutant is a corresponding truncated form of IL-2 having a wild-type sequence. Illustratively, the "wild-type IL-2" described herein may have the amino acid sequence shown below: APTSSSTKKTQLQLEHLLLDLQMILNGINNYKNPKLTRMLTFKFYMPKKATELKHLQCLEE ELKPLEEVLNLAQSKNFHLRPRDLISNINVIVLELKGSETTFMCEYADETATIVEFLNRWITF**X** QSIISTLT (SEQ ID NO: 1); wherein the amino acid residue "X" at position 125 represents C, S, A, or V

The term "mutation" of the present invention encompasses amino acid substitutions, deletions, insertions, or any combination thereof. The "mutation" described herein may be generated by using genetic or chemical methods known in the art, including but not limited to, site-directed mutagenesis, PCR, gene synthesis, and the like.

"Mutation sites" of the IL-2 mutant described herein are numbered from amino acid residue A at position 1 of the "wild-type IL-2" set forth in SEQ ID NO: 1. Illustratively, the "Y31 mutation" of the present invention means that the IL-2 mutant has a mutation of the amino acid residue at position 31 (Tyr, Y) of the wild-type IL-2 set forth in SEQ ID NO: 1. Illustratively, the "Y31V mutation" of the present invention means that the IL-2 mutant has a mutation of the amino acid residue from Y (Tyr) to V (Val) at position 31 of the wild-type IL-2 set forth in SEQ ID NO: 1.

The term "/" used herein between mutation sites indicates "and", and means that mutations before and after "j" are present in the same IL-2 mutant at the same time. Illustratively, "Y31/A73/H79" means that mutations occur at sites Y31, A73, and H79 in the same IL-2 mutant at the same time, and "Y31V/A73L/H79Q" means that mutations Y31V, A73L, and H79Q are present in the same IL-2 mutant at the same time.

The term "fusion protein" in the present invention refers to a protein product obtained by gene recombination, which is expressed under the control of the same regulatory sequence by linking the coding regions of two or more genes by gene recombination method, chemical method, or other appropriate methods. In the fusion protein of the present invention, the coding regions of two or more genes may be fused at one or several positions by the sequence encoding a linker peptide. Linker peptides may also be used to construct the fusion protein of the present invention.

The term "linker" in the present invention refers to a peptide used in the present invention to link IL-2 to another protein molecule or protein fragment to ensure proper folding and stability of the protein. The another molecule includes, but is not limited to, an antibody Fc block. The "linker" of the present invention is preferably (GGGGS (SEQ ID NO: 21))n, where n may be 0, 1, 2, 3, 4, 5, or 6.

The term "antibody Fc block" in the present invention refers to a constant region of an immunoglobulin chain, particularly the carboxy-terminus of an immunoglobulin heavy chain constant region or a portion thereof, which has no antigen-binding activity and is the site of interaction of an antibody molecule with an effector molecule or a cell. The "Fc" described herein may be any Fc or a variant thereof, derived from a human or non-human mammal. For example, the immunoglobulin Fc may comprise a combination of two or more of heavy chain CH1, CH2, CH3, and CH4 domains with an immunoglobulin hinge region. The Fc may be derived from a different species, preferably a human immunoglobulin. Based on the amino acid sequence of the heavy chain constant region, immunoglobulins may be divided into different types. There are mainly 5 types of immunoglobulins: IgA, IgD, IgE, IgG, and IgM. Some of them may be further divided into subtypes (isoforms), such as IgG-1, IgG-2, IgG-3, IgG-4, IgA-1, and IgA-2. The "Fc" preferably comprises at least one immunoglobulin hinge region, and CH2 and CH3 domains of IgG. More preferably, it comprises one CH2 domain, one CH3 domain, and one immunoglobulin hinge region of IgG1. The starting amino acid position of the hinge region may vary. Unless otherwise indicated, amino acid residues in the Fc, constant regions, or antibodies described herein are numbered according to the EU numbering system, also known as the EU index, as described in Kabat et al., Sequences of Proteins of Immunological Interes, 5th Ed, Public Health Service, National Institutes of Health, Bethesda, MD, 1991.

The term "pharmaceutical composition" in the present invention refers to a mixture comprising one or more of the IL-2 mutants, fusion proteins, nucleic acid fragments, vectors, or host cells described herein. The mixture further comprises other components, including but not limited to, pharmaceutically acceptable carriers, diluents, or adjuvants thereof. The pharmaceutical composition described herein is intended to promote the administration to an organism, facilitate the absorption of active ingredients, and thereby exert biological activity.

The term "treatment" in the present invention refers to surgical or therapeutic treatment for the purpose of preventing or slowing (reducing) the progression of an undesired physiological or pathological change, e.g., an autoimmune disease (e.g., systemic lupus erythematosus), in a subject being treated. Beneficial or desired clinical results include, but are not limited to, alleviation of symptoms, a decrease of severity of disease, stabilization (i.e., not worsening) of state of disease, delay or slowing of disease progression, amelioration or palliation of state of disease, and remission (whether partial or total) of state of disease, whether detectable or undetectable. Subjects in need of treatment include those already with a disorder or disease, as well as those who are susceptible to a disorder or disease or those who intend to prevent a disorder or disease. When referring to terms such as slowing, alleviation, decrease, palliation, and remission, their meanings also include elimination, disappearance, nonoccurrence, etc.

The term "subject" in the present invention refers to an organism that receives treatment for a particular disease or disorder (e.g., an autoimmune disease) described herein. Examples of subjects and patients include mammals, such as humans, primates, pigs, goats, rabbits, hamsters, cats, dogs, guinea pigs, cows or other members of the bovid family, sheep, horses, etc., that receive treatment for a disease or disorder.

The term "effective amount" in the present invention refers to an amount of a therapeutic agent that is effective in preventing or alleviating symptoms of a disease or the progression of the disease when administered to a cell, tissue, or subject alone or in combination with another therapeutic agent. "Effective amount" also refers to an amount of a compound that is sufficient to alleviate symptoms, e.g., to treat, cure, prevent, or alleviate related medical disorders, or to increase the rates at which such disorders are treated, cured, prevented, or alleviated. When the active ingredient is administered alone to an individual, a therapeutically effective dose refers to the amount of the ingredient alone. When a combination is used, a therapeutically effective dose refers to the combined amounts of the active ingredients that produce the therapeutic effect, whether administered in combination, sequentially, or simultaneously.

The term "autoimmune disease" in the present invention refers to a disorder in a subject characterized by cellular, tissue and/or organ damage resulting from an immune response in the subject to its own cells, tissues and/or organs. Illustratively, the autoimmune disease includes, but is not limited to, rheumatoid arthritis, ankylosing spondylitis, systemic lupus erythematosus, cutaneous lupus erythematosus, lupus nephritis, IgA nephropathy, Sjogren's syndrome, polymyositis, dermatomyositis, scleroderma, psoriasis, plaque psoriasis, alopecia areata, multiple sclerosis, amyotrophic lateral sclerosis, inflammatory bowel disease, ulcerative colitis, Crohn's disease, graft-versus-host disease, organ transplant rejection, autoimmune hepatitis, type I diabetes, autoimmune vasculitis, eczema, or asthma.

The term "proliferative disease" in the present invention refers to a condition in which the growth of cells or tissues is unregulated and/or abnormal, which may lead to the development of undesired conditions or diseases. The proliferative disease may or may not be cancerous, including but not limited to, neoplasms, solid tumors, hematologic tumors, malignant ascites, or malignant pleural effusion. The "solid tumor" in the present invention may be benign or malignant, primary or metastatic; malignant solid tumors may be carcinomas or sarcomas. Illustratively, the "solid tumor" in the present invention includes, but is not limited to, epithelial cell carcinoma, endothelial cell carcinoma, squamous cell carcinoma, teratoma, lung tumor, papillomavirus-induced cancer, adenocarcinoma, carcinoma, melanoma, angiosarcoma, neuroblastoma, metastatic lung cancer, non-small cell lung cancer, small cell lung cancer, breast cancer, Merkel cell carcinoma, ovarian cancer, renal cell carcinoma, metastatic renal cancer, head and neck cancer, bladder cancer, and non-muscle invasive bladder cancer. Illustratively, the "hematologic tumor" in the present invention includes, but is not limited to, leukemia, lymphoma, and multiple myeloma, such as B-cell lymphoma, T-cell lymphoma, cutaneous T-cell lymphoma, and T-cell large granular lymphocytic leukemia.

In the present invention, the term "IL-2 receptor α subunit" (IL-2Rα), also known as "CD25", refers to any natural IL-2 receptor α subunits or mutants thereof derived from any vertebrates, including mammals such as primates (e.g., humans) and rodents (e.g., mice and rats). The term includes "full-length" unprocessed IL-2 receptor α subunits as well as any form of processed IL-2 receptor α subunits derived from cells, also includes naturally occurring IL-2 receptor α subunit variants, such as splice variants or allelic variants, and further includes artificially engineered mutants on the basis of the natural IL-2 receptor α subunits.

In the present invention, the term "IL-2 receptor β subunit" (IL-2Rβ), also known as "CD122", refers to any natural IL-2 receptor β subunits or mutants thereof derived from any vertebrates, including mammals such as primates (e.g., humans) and rodents (e.g., mice and rats). The term includes "full-length" unprocessed IL-2 receptor β subunits as well as any form of processed IL-2 receptor β subunits derived from cells, also includes naturally occurring IL-2 receptor β subunit variants, such as splice variants or allelic variants, and further includes artificially engineered mutants on the basis of the natural IL-2 receptor β subunits.

In the present invention, the term "IL-2 receptor γ subunit" (IL-2Rγ), also known as "CD132", refers to any natural IL-2 receptor γ subunits or mutants thereof derived from any vertebrates, including mammals such as primates (e.g., humans) and rodents (e.g., mice and rats). The term includes "full-length" unprocessed IL-2 receptor γ subunits as well as any form of processed IL-2 receptor γ subunits derived from cells, also includes naturally occurring IL-2 receptor γ subunit variants, such as splice variants or allelic variants, and further includes artificially engineered mutants on the basis of the natural IL-2 receptor γ subunits.

In the present invention, the term "Treg", also known as "regulatory T cell" or "T_{regulatory cell}", refers to a specialized CD4⁺ T cell type that can inhibit the response of other T cells. The Treg is characterized by the expression of the IL-2 receptor α subunit (CD25) and the transcription factor forkhead box protein P3 (FOXP3), and plays a key role in the induction and maintenance of peripheral self-tolerance to antigens. The Treg requires IL-2 for its function and development as well as for the induction of its inhibitory characteristics.

As used herein, the terms "percent (%) sequence consistency" and "percent (%) sequence identity" are used interchangeably, referring to the percentage of identity between amino acid (or nucleotide) residues of a candidate sequence and amino acid (or nucleotide) residues of a reference sequence after aligning the sequences and introducing gaps, if necessary, to achieve maximum percent sequence identity (e.g., gaps may be introduced in one or both of the candidate sequence and the reference sequence for optimal alignment, and non-homologous sequences may be omitted for the purpose of comparison). Alignment may be carried out in a variety of ways well known to those skilled in the art for the purpose of determining percent sequence identity, for example, using publicly available computer software such as BLAST, ALIGN, or Megalign (DNASTAIi) software. Those skilled in the art can determine appropriate parameters for measuring alignment, including any algorithms required to achieve maximum alignment of the full length of the aligned sequences. For example, a reference sequence aligned for comparison with a candidate sequence may show that the candidate sequence exhibits 50% to 100% sequence identity over the full length of the candidate sequence or a selected portion of contiguous amino acid (or nucleotide) residues of the candidate sequence. The length of the candidate sequence aligned for the purpose of comparison may be, e.g., at least 30% (e.g., 30%, 40%, 50%, 60%, 70%, 80%, 90%, or 100%) of the length of the reference sequence. When a position in the candidate sequence is occupied by the same amino acid (or nucleotide) residue at the corresponding position in the reference sequence, then the molecules are identical at that position.

### BRIEF DESCRIPTION OF THE DRAWING

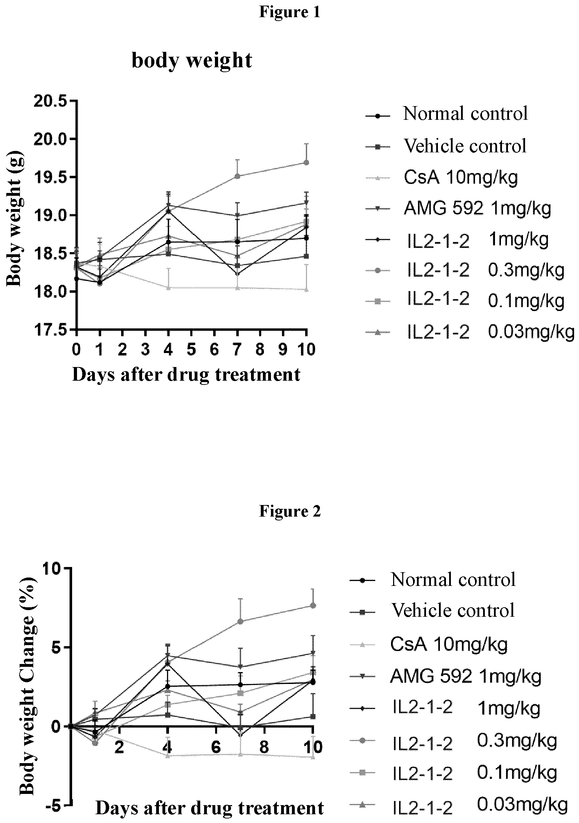

FIG. 1 shows body weight change curves of mice, specifically body weight change curves of mice after administration of CsA, AMG592, and IL2-1-2 injections to DTH model mice, 10 mice per group, wherein data points represent the mean body weight of animals in a group, and error bars represent standard error (SEM).
FIG. 2 shows body weight change rate curves of mice, specifically body weight change rates of DTH model mice after administration of CsA, AMG592, and IL2-1-2 injections, 10 mice per group, wherein data points represent the mean body weight change rate of animals in a group, and error bars represent standard error (SEM).

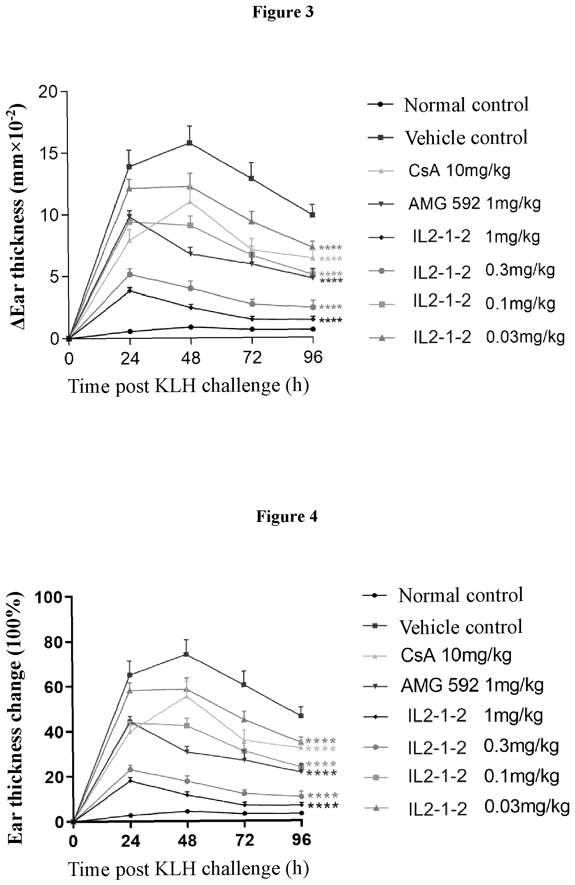

FIG. 3 shows ear thickness change curves of DTH model mice, specifically ear thickness change curves of DTH model mice after administration of CsA, AMG592, and IL2-1-2 injections, 10 mice per group, wherein data points represent the mean ear thickness change value in a group, and error bars represent standard error (SEM). **** P < 0.0001 as compared to the vehicle control group.
FIG. 4 shows ear thickness change rate curves after administration of CsA, AMG592, and IL2-1-2 injections to DTH model mice, 10 mice per group, wherein data points represent the mean ear thickness change value in a group, and error bars represent standard error (SEM). **** P < 0.0001 as compared to the vehicle control group.

### DETAILED DESCRIPTION

The present invention will be further described with reference to specific examples, and the advantages and features of the present invention will become more apparent with the description. Experimental procedures without specified conditions in the examples are conducted according to conventional conditions or conditions recommended by the manufacturers. Reagents or instruments without specified manufacturers used herein are conventional products that are commercially available.

The examples are exemplary only and do not limit the scope of the present invention in any way. It will be understood by those skilled in the art that various changes or substitutions in form and details may be made to the technical solutions of the present invention without departing from the spirit and scope of the present invention, and that these changes and substitutions shall fall within the scope of the present invention.

### Description of Examples 1-5

### 1. Buffer exchange by ultrafiltration concentration

Unless otherwise specified, the buffer exchange by ultrafiltration concentration in the following examples refers to: adding a protein stock solution to a corresponding ultrafiltration tube; performing ultrafiltration concentration by using a centrifuge; retaining proteins; enabling a buffer of the protein stock solution to flow through the ultrafiltration membrane to achieve the purpose of protein concentration; adding a target solution to be exchanged, and diluting the original buffer of the protein stock solution; performing ultrafiltration concentration again; adding the target solution to be exchanged again after completion of the concentration; performing ultrafiltration concentration again; and repeating these operations until the buffer exchange is completed.

### 2. English abbreviations and meanings thereof

Unless otherwise specified, the following English abbreviations have the meanings shown in the table below:

**Table 1. Description of abbreviations**

| Abbreviation | Meaning |
|---|---|
| T0 | Representing "starting point of investigation" |
| W | Representing "week", e.g., "1 W" represents "1 week" |
| M | Representing "month", e.g., "3 M" represents "3 months" |
| C | Representing cycle number of freezing-thawing, e.g., "3C" represents 3 cycles of freezing-thawing, and "5C" represents 5 cycles of freezing-thawing |
| D | Representing "day", e.g., "7 D" represents "7 days" |
| H | Representing "hour" |
| ND | Representing "not detected" |
| NA | Representing "not applicable" |
| SEC-HPLC | Size-exclusion high-performance liquid chromatography |
| CE-SDS (NR) | Non-reduced capillary electrophoresis-sodium dodecyl sulfate |
| CE-SDS (R) | Reduced capillary electrophoresis-sodium dodecyl sulfate |
| iCIEF | Whole-column imaging capillary isoelectric focusing |
| DLS | Dynamic light scattering |
| DSF | Differential scanning fluorimetry |

### 3. Assay method for binding activity of IL2 mutant fusion protein in Example 5

(1) Coating: A carbonate buffer (50 mM Na₂CO₃-NaHCO₃, pH 9.6) with a pH of 9.6 was selected as a coating solution; human IL-2R alpha protein (Aero, ILA-H52H9) was diluted to 0.5 µg/mL with the coating solution; and the mixture was added to an ELISA plate at 100 µL/well and coated at 2-8 °C overnight (> 16 h).
(2) Blocking: After the plate was washed, PBS containing 3% BSA was added to the plate at 300 µL/well, and the plate was blocked at 37 °C for 1 h and then washed.
(3) Sample dilution: The IL2 mutant fusion protein was diluted from a starting concentration of 200 ng/mL (see Table 2 for details).

**Table 2. Dilution method for IL2 mutant fusion protein**

| Sample No. | Sample concentration (ng/mL) | Sample volume (µL) | Diluent volume (µL) | Total volume (µL) | Remaining volume (µL) |
|---|---|---|---|---|---|
| Sample 1 | 200 | / | / | / | / |
| Sample 2 | 60.06006 | 120 (sample 1) | 280 | 400 | 280 |
| Sample 3 | 18.03605 | 120 (sample 2) | 280 | 400 | 280 |
| Sample 4 | 5.416232 | 120 (sample 3) | 280 | 400 | 280 |
| Sample 5 | 1.626496 | 120 (sample 4) | 280 | 400 | 280 |
| Sample 6 | 0.488437 | 120 (sample 5) | 280 | 400 | 280 |
| Sample 7 | 0.14667 | 120 (sample 6) | 280 | 400 | 280 |
| Sample 8 | 0.044047 | 120 (sample 7) | 280 | 400 | 400 |

| | | | | | |
|---|---|---|---|---|---|
| Note: 120 µL of the prepared sample 1 was taken, 280 µL of the sample diluent was added, and the resulting mixture was mixed well to give sample 2; 120 µL of the prepared sample 2 was taken, 280 µL of the sample diluent was added, and the resulting mixture was mixed well to give sample 3. Samples 4-8 were diluted in the same way. | | | | | |

(4) The diluted sample of the IL2 mutant fusion protein was added at 100 µL/well, and the mixture was incubated at 37 °C for 40-80 min.
(5) After the mixture was washed, mouse anti-human IgG Fc γ-HRP (Jackson Immuno, 209-035-098) diluted at 1:10000 (v/v) was added at 100 µL/well, and the resulting mixture was incubated at 37 °C for 40-80 min.
(6) After the mixture was washed, a color development solution TMB (Thermo, 34029) was added to the ELISA plate at 100 µL/well; the resulting mixture was reacted at room temperature for 5-9 min in the dark, and then 1 M sulfuric acid was added at 100 µL/well to stop the reaction; and the plate was read within 2 min using a microplate reader, at a measurement wavelength of 450 nm and a reference wavelength of 630 nm.
(7) EC50 values of the test samples and the control were calculated by four-parameter regression using the SoftMax Pro or GraphPad Prism software provided by the Molecular Devices microplate reader or the same type of analysis software. The relative activity of the test sample was calculated according to the following formula: relative binding activity of the test sample (%) = EC50 of the control/EC50 of the test sample × 100. The test sample was determined to be qualified when the relative activity of the test sample was in the range of 70%-130%.

### Example 1. Preparation and Purification of IL2 Mutant Fusion Proteins

### 1. Design of IL2 mutant fusion proteins

(1) Mutation sites that could improve the thermal stability of IL2, Y31V/A73L/H79Q, were designed using various algorithms.
(2) Functional mutation sites that could attenuate the interaction of IL2 with the IL2 receptor βγ subunit complex, V91R, H16E, D20A, or H16E/V91R, were designed using various algorithms.
(3) The thermostable mutations and functional mutations described above were combined to obtain IL2 mutants comprising the following combinatorial mutations:
   Y31V/A73L/H79Q/V91R;
   Y31V/A73L/H79Q/H16E;
   Y31V/A73L/H79Q/D20A; and
   Y31V/A73L/H79Q/H16E/V91R.

In order to prolong the half-life, the thermostable mutant or combinatorial mutant of IL2 was further linked to Fc via a linker to construct a fusion protein (hereinafter referred to as IL2 mutant fusion protein). The elements and their overall sequences are shown in Table 3.

Compared with the wild-type IL2 fusion protein (SEQ ID NO: 19), the IL2 mutant fusion protein described herein has the following characteristics:
(1) Compared with the wild-type IL2 fusion protein, the Tm values of the IL2 mutant fusion proteins containing the thermostable mutations were increased by analysis of a DSF method: the Tm value of the IL2 mutant 1-linker-Fc was increased by about 8-9 °C, the Tm value of the IL2 mutant 1-2-linker-Fc was increased by 12 °C or more, the Tm value of the IL2 mutant 1-3-linker-Fc was increased by 8-9 °C, the Tm value of the IL2 mutant 1-4-linker-Fc was increased by about 9-10 °C, and the Tm value of the IL2 mutant 1-5-linker-Fc was increased by about 11-12 °C.
(2) Compared with the wild-type IL2 fusion protein, the IL2 mutant fusion proteins containing the functional mutations had inhibited binding activity to the human IL2 receptor βγ dimer on the cell surface, the activation of STATS phosphorylation levels in CD4⁺ CD25⁻ FoxP3⁻ T cells or CD8⁺ T cells was significantly reduced, the effects of the IL2 mutant fusion proteins on the proliferation of Treg cells were similar to that of the wild-type IL2 fusion protein, and the proliferation level of NK cells was significantly reduced.
(3) Compared with the wild-type IL2 fusion protein or Fc-linker-IL2 mutant 6, the IL2 mutant 1-2-linker-Fc showed better efficacy in terms of pharmaceutical effects (DTH model mice and GVHD model mice), higher exposure and bioavailability in terms of pharmacokinetics, and longer half-life, which were more significant after subcutaneous administration.
(4) The IL2 mutant 1-2-linker-Fc (Y31V/A73L/H79Q/V91R, SEQ ID NO: 15) was selected for subsequent process development.

**Table 3. Sequence information of IL2 mutant fusion proteins**

| Name | | Sequence |
|---|---|---|
| Wild type | IL2 | APTSSSTKKTQLQLEHLLLDLQMILNGINNYKNPKLTRMLTFKF YMPKKATELKHLQCLEEELKPLEEVLNLAQSKNFHLRPRDLIS NINVIVLELKGSETTFMCEYADETATIVEFLNRWITFAQSIISTLT (SEQ ID NO:2) |
| Thermostable mutation | IL2 mutant 1 (Y31V/A73L/H79 Q) | APTSSSTKKTQLQLEHLLLDLQMILNGINNVKNPKLTRMLTFKF YMPKKATELKHLQCLEEELKPLEEVLNLLQSKNFQLRPRDLIS NINVIVLELKGSETTFMCEYADETATIVEFLNRWITFAQSIISTLT (SEQ ID NO:3) |
| Functional mutation | IL2 mutant 2 (V91R) | APTSSSTKKTQLQLEHLLLDLQMILNGINNYKNPKLTRMLTFKF YMPKKATELKHLQCLEEELKPLEEVLNLAQSKNFHLRPRDLIS NINRIVLELKGSETTFMCEYADETATIVEFLNRWITFAQSIISTLT (SEQ ID NO:4) |
| | IL2 mutant 3 (H16E) | APTSSSTKKTQLQLEELLLDLQMILNGINNYKNPKLTRMLTFKF YMPKKATELKHLQCLEEELKPLEEVLNLAQSKNFHLRPRDLIS NINVIVLELKGSETTFMCEYADETATIVEFLNRWITFAQSIISTLT (SEQ ID NO:5) |
| | IL2 mutant 4 (D20A) | APTSSSTKKTQLQLEHLLLALQMILNGINNYKNPKLTRMLTFKF YMPKKATELKHLQCLEEELKPLEEVLNLAQSKNFHLRPRDLIS NINVIVLELKGSETTFMCEYADETATIVEFLNRWITFAQSIISTLT (SEQ ID NO:6) |
| | IL2 mutant 5 (H16E/V91R) | APTSSSTKKTQLQLEELLLDLQMILNGINNYKNPKLTRMLTFKF YMPKKATELKHLQCLEEELKPLEEVLNLAQSKNFHLRPRDLIS NINRIVLELKGSETTFMCEYADETATIVEFLNRWITFAQSIISTLT (SEQ ID NO:7) |
| Combinatorial mutation | IL2 mutant 1-2 (Y31V/A73L/H79 Q/V91R) | APTSSSTKKTQLQLEHLLLDLQMILNGINNVKNPKLTRMLTFKF YMPKKATELKHLQCLEEELKPLEEVLNLLQSKNFQLRPRDLIS NINRIVLELKGSETTFMCEYADETATIVEFLNRWITFAQSIISTLT (SEQ ID NO:8) |
| | IL2 mutant 1-3 (H16E/Y31V/A73 L/H79Q) | APTSSSTKKTQLQLEELLLDLQMILNGINNVKNPKLTRMLTFKF YMPKKATELKHLQCLEEELKPLEEVLNLLQSKNFQLRPRDLIS NINVIVLELKGSETTFMCEYADETATIVEFLNRWITFAQSIISTLT (SEQ ID NO:9) |
| | IL2 mutant 1-4 (D20A/Y31V/A73 | APTSSSTKKTQLQLEHLLLALQMILNGINNVKNPKLTRMLTFKF YMPKKATELKHLQCLEEELKPLEEVLNLLQSKNFQLRPRDLIS |
| | L/H79Q) | NINVIVLELKGSETTFMCEYADETATIVEFLNRWITFAQSIISTLT (SEQ ID NO:10) |
| | IL2 mutant 1-5 (H16E/V91R/Y31 V/A73L/H79Q) | APTSSSTKKTQLQLEELLLDLQMILNGINNVKNPKLTRMLTFKF YMPKKATELKHLQCLEEELKPLEEVLNLLQSKNFQLRPRDLIS NINRIVLELKGSETTFMCEYADETATIVEFLNRWITFAQSIISTLT (SEQ ID NO:11) |
| Linker | | GGGGSGGGGSGGGGS (SEQ ID NO:12) |
| Fc | | EPKSSDKTHTCPPCPAPELLGGPSVFLFPPKPKDTLMISRTPEVT CVVVDVSHEDPEVKFNWYVDGVEVHNAKTKPREEQYGSTYR VVSVLTVLHQDWLNGKEYKCKVSNKALPAPIEKTISKAKGQPR EPQVYTLPPSRDELTKNQVSLTCLVKGFYPSDIAVEWESNGQPE NNYKTTPPVLDSDGSFFLYSKLTVDKSRWQQGNVFSCSVMHE ALHNHYTQKSLSLSPGK (SEQ ID NO:13) |
| IL2 mutant 1-linker-Fc (Y31V/A73L/H79Q) | | APTSSSTKKTQLQLEHLLLDLQMILNGINNVKNPKLTRMLTFKF YMPKKATELKHLQCLEEELKPLEEVLNLLQSKNFQLRPRDLIS NINVIVLELKGSETTFMCEYADETATIVEFLNRWITFAQSIISTLT GGGGSGGGGSGGGGSEPKSSDKTHTCPPCPAPELLGGPSVFL FPPKPKDTLMISRTPEVTCVVVDVSHEDPEVKFNWYVDGVEV HNAKTKPREEQYGSTYRVVSVLTVLHQDWLNGKEYKCKVSN KALPAPIEKTISKAKGQPREPQVYTLPPSRDELTKNQVSLTCLVK GFYPSDIAVEWESNGQPENNYKTTPPVLDSDGSFFLYSKLTVDK SRWQQGNVFSCSVMHEALHNHYTQKSLSLSPGK (SEQ ID NO:14) |
| IL2 mutant 1-2-linker-Fc (V91R/Y31V/A73L/H79Q) | | APTSSSTKKTQLQLEHLLLDLQMILNGINNVKNPKLTRMLTFKF YMPKKATELKHLQCLEEELKPLEEVLNLLQSKNFQLRPRDLIS NINRIVLELKGSETTFMCEYADETATIVEFLNRWITFAQSIISTLT GGGGSGGGGSGGGGSEPKSSDKTHTCPPCPAPELLGGPSVFL FPPKPKDTLMISRTPEVTCVVVDVSHEDPEVKFNWYVDGVEV HNAKTKPREEQYGSTYRVVSVLTVLHQDWLNGKEYKCKVSN KALPAPIEKTISKAKGQPREPQVYTLPPSRDELTKNQVSLTCLVK GFYPSDIAVEWESNGQPENNYKTTPPVLDSDGSFFLYSKLTVDK SRWQQGNVFSCSVMHEALHNHYTQKSLSLSPGK (SEQ ID NO:15) |
| IL2 mutant 1-3-linker-Fc (H16E/Y31V/A73L/H79Q) | | APTSSSTKKTQLQLEELLLDLQMILNGINNVKNPKLTRMLTFKF YMPKKATELKHLQCLEEELKPLEEVLNLLQSKNFQLRPRDLIS NINVIVLELKGSETTFMCEYADETATIVEFLNRWITFAQSIISTLT GGGGSGGGGSGGGGSEPKSSDKTHTCPPCPAPELLGGPSVFL FPPKPKDTLMISRTPEVTCVVVDVSHEDPEVKFNWYVDGVEV HNAKTKPREEQYGSTYRVVSVLTVLHQDWLNGKEYKCKVSN KALPAPIEKTISKAKGQPREPQVYTLPPSRDELTKNQVSLTCLVK GFYPSDIAVEWESNGQPENNYKTTPPVLDSDGSFFLYSKLTVDK SRWQQGNVFSCSVMHEALHNHYTQKSLSLSPGK (SEQ ID NO:16) |
| IL2 mutant 1-4-linker-Fc (D20A/Y31V/A73L/H79Q) | | APTSSSTKKTQLQLEHLLLALQMILNGINNVKNPKLTRMLTFKF YMPKKATELKHLQCLEEELKPLEEVLNLLQSKNFQLRPRDLIS NINVIVLELKGSETTFMCEYADETATIVEFLNRWITFAQSIISTLT GGGGSGGGGSGGGGSEPKSSDKTHTCPPCPAPELLGGPSVFL |
| | | FPPKPKDTLMISRTPEVTCVVVDVSHEDPEVKFNWYVDGVEV HNAKTKPREEQYGSTYRVVSVLTVLHQDWLNGKEYKCKVSN KALPAPIEKTISKAKGQPREPQVYTLPPSRDELTKNQVSLTCLVK GFYPSDIAVEWESNGQPENNYKTTPPVLDSDGSFFLYSKLTVDK SRWQQGNVFSCSVMHEALHNHYTQKSLSLSPGK (SEQ ID NO:17) |
| IL2 mutant 1-5-linker-Fc (H16E/V91R/Y31V/A73L/H79Q) | | APTSSSTKKTQLQLEELLLDLQMILNGINNVKNPKLTRMLTFKF YMPKKATELKHLQCLEEELKPLEEVLNLLQSKNFQLRPRDLIS NINRIVLELKGSETTFMCEYADETATIVEFLNRWITFAQSIISTLT GGGGSGGGGSGGGGSEPKSSDKTHTCPPCPAPELLGGPSVFL FPPKPKDTLMISRTPEVTCVVVDVSHEDPEVKFNWYVDGVEV HNAKTKPREEQYGSTYRVVSVLTVLHQDWLNGKEYKCKVSN KALPAPIEKTISKAKGQPREPQVYTLPPSRDELTKNQVSLTCLVK GFYPSDIAVEWESNGQPENNYKTTPPVLDSDGSFFLYSKLTVDK SRWQQGNVFSCSVMHEALHNHYTQKSLSLSPGK (SEQ ID NO:18) |
| IL2-linker-Fc | | APTSSSTKKTQLQLEHLLLDLQMILNGINNYKNPKLTRMLTFKF YMPKKATELKHLQCLEEELKPLEEVLNLAQSKNFHLRPRDLIS NINVIVLELKGSETTFMCEYADETATIVEFLNRWITFAQSIISTLT GGGGSGGGGSGGGGSEPKSSDKTHTCPPCPAPELLGGPSVFL FPPKPKDTLMISRTPEVTCVVVDVSHEDPEVKFNWYVDGVEV HNAKTKPREEQYGSTYRVVSVLTVLHQDWLNGKEYKCKVSN KALPAPIEKTISKAKGQPREPQVYTLPPSRDELTKNQVSLTCLVK GFYPSDIAVEWESNGQPENNYKTTPPVLDSDGSFFLYSKLTVDK SRWQQGNVFSCSVMHEALHNHYTQKSLSLSPGK (SEQ ID NO:19) |
| Fc-linker-IL2 mutant 6 (V91K) | | PKSSDKTHTCPPCPAPELLGGPSVFLFPPKPKDTLMISRTPEVTC VVVDVSHEDPEVKFNWYVDGVEVHNAKTKPCEEQYGSTYRC VSVLTVLHQDWLNGKEYKCKVSNKALPAPIEKTISKAKGQPRE PQVYTLPPSRDELTKNQVSLTCLVKGFYPSDIAVEWESNGQPEN NYKTTPPVLDSDGSFFLYSKLTVDKSRWQQGNVFSCSVMHEA LHNHYTQKSLSLSPGGGGGSAPTSSSTKKTQLQLEHLLLDLQ MILNGINNYKNPKLTRMLTFKFYMPKKATELKHLQCLEEELKP LEEVLNLAQSKNFHLRPRDLISNINKIVLELKGSETTFMCEYAD ETATIVEFLNRWITFAQSIISTLT (SEQ ID NO:20) |

### 2. Expression of IL2 mutant fusion proteins

The IL2 mutant fusion protein was expressed by using CHO cells as a host cell, and was cultured in an OPM-CHO CD063 medium (manufacturer: OPM, Catalog No. C483260). The culturing was performed for no more than 14 days. The reactor control parameters were as follows: pH: 6.8-7.2; dissolved oxygen (DO): 20%-80%; rotation speed: 75 RPM-80 RPM; and initial culture temperature: 36.0 °C-37.0 °C. When the fusion proteins were cultured to day 6, the culture temperature was reduced to 34.0±0.5 °C until the harvest.

### 3. Purification of IL2 mutant fusion proteins

The IL2 mutant fusion protein was sequentially purified by using multi-step chromatography, concentration, and filtration unit operations: the fermentation harvest supernatant was captured by Protein A affinity chromatography (AT ProteinA Diamond Plus). The captured IL2 mutant fusion protein solution was treated with low-pH incubation to inactivate potential viruses. The precipitate was removed by depth filtration after neutralization of the IL2 mutant fusion protein solution. Cation exchange chromatography (Diamond S) was performed to remove impurities such as HCP and aggregates, and then anion exchange chromatography (POROS 50HQ) was performed to remove impurities such as HCD, HCP, and shed Protein A. The fusion protein solution was filtered through a nanofiltration membrane to remove potential endogenous and exogenous viruses. Then, the IL2 mutant fusion protein solution was concentrated and subjected to buffer exchange with an ultrafiltration membrane. Finally, an excipient was added, the concentration was regulated, and the resulting mixture was filtered to give a stock solution of the IL2 mutant fusion protein. In the following examples, the stock solution was exchanged to the corresponding formulation system by ultrafiltration concentration and buffer exchange treatment.

### Examples 2-5. Development of Formulation Formulas to Maintain Stability of IL2 Mutant 1-2-Linker-Fc (V91R/Y31V/A73L/H79Q, SEQ ID NO: 15) in Example 1

### Example 2. Buffer System Screening

### 1. Screening protocol

(1) Twelve different buffer formula systems F1-F12 were designed. The specific formula systems are shown in Table 4. (2) The stock solution of the IL2 mutant fusion protein obtained in Example 1 was exchanged to F 1-F 12 by ultrafiltration concentration and buffer exchange treatment. The protein concentration was adjusted to 2 mg/mL. (3) Through accelerated stability tests at 25 °C and accelerated stability tests at 40 °C, the stability of the IL2 mutant fusion protein in 12 buffer systems was comprehensively investigated to select an optimal buffer system for subsequent screening of excipients and surfactants. The investigation indexes include appearance, pH value, protein concentration, purity (SEC-HPLC), CE-SDS (NR), CE-SDS (R), and dynamic light scattering (DLS). The investigation protocol is detailed in Table 4.

**Table 4. Formulation buffer systems and screening investigation protocol**

| Formula No. | Buffer system, pH | | T0 | 25°C | | 40°C | | |
|---|---|---|---|---|---|---|---|---|
| | | | | 2W | 4W | 1W | 2W | 4W |
| F1 | 20 mM acetic acid-sodium acetate | 4.5 | X | X | X | X | X | X |
| F2 | | 5.0 | X | X | X | X | X | X |
| F3 | | 5.5 | X | X | X | X | X | X |
| F4 | 20 mM citric acid-sodium citrate | 5.0 | X | X | X | X | X | X |
| F5 | | 5.5 | X | X | X | X | X | X |
| F6 | | 6.0 | X | X | X | X | X | X |
| F7 | 20 mM histidine-histidin e hydrochloride | 5.5 | X | X | X | X | X | X |
| F8 | | 6.0 | X | X | X | X | X | X |
| F9 | | 6.5 | X | X | X | X | X | X |
| F10 | 20 mM disodium hydrogen phosphate-sodiu m dihydrogen phosphate | 6.5 | X | X | X | X | X | X |
| F11 | | 7.0 | X | X | X | X | X | X |
| F12 | | 7.5 | X | X | X | X | X | X |

| | | | | | | | | |
|---|---|---|---|---|---|---|---|---|
| Note: X = appearance, pH, protein concentration, DLS, SEC-HPLC, and CE-SDS (NR & R). | | | | | | | | |

### 2. Buffer system screening results

The major results of the buffer system screening are summarized below:

**Table 5. Appearance detection results of formulation formulas in accelerated stability tests at 25 °C or 40 °C**

| Formula No. | T0 | 25°C-2W | 25°C-4W | 40°C-4W |
|---|---|---|---|---|
| F1 | Colorless, clear | Colorless, clear | Colorless, clear | Colorless, clear, with a small amount of flocs |
| F2 | Colorless, clear | Colorless, clear | Colorless, clear, with a small amount of flocs | Colorless, clear, with a small amount of flocs |
| F3 | Colorless, clear | Colorless, clear | Colorless, clear | Colorless, clear, with a small amount of flocs |
| F4 | Colorless, clear, with a small amount of flocs | Colorless, clear, with a small amount of flocs | Colorless, clear, with a small amount of flocs | Colorless, clear, with more flocs |
| F5 | Colorless, clear, with a small amount of flocs | Colorless, clear, with a small amount of flocs | Colorless, clear, with a small amount of flocs | Colorless, clear, with more flocs |
| F6 | Colorless, clear, with a small amount of flocs | Colorless, clear, with a small amount of flocs | Colorless, clear, with a small amount of flocs | Colorless, clear, with a small amount of flocs |
| F7 | Colorless, clear | Colorless, clear | Colorless, clear, with a small amount of flocs | Colorless, clear, with a small amount of flocs |
| F8 | Colorless, clear | Colorless, clear | Colorless, clear, with a small amount of flocs | Colorless, clear, with a small amount of flocs |
| F9 | Colorless, clear | Colorless, clear | Colorless, clear, with a small amount of flocs | Colorless, clear, with a small amount of flocs |
| F10 | Colorless, clear | Colorless, clear, with a small amount of flocs | Colorless, clear, with a small amount of flocs | Colorless, clear, with a small amount of flocs |
| F11 | Colorless, clear | Colorless, clear | Colorless, clear, with a small amount of flocs | Colorless, clear, with a small amount of flocs |
| F12 | Colorless, clear | Colorless, clear, with a small amount of flocs | Colorless, clear, with a small amount of flocs | Colorless, clear, with a small amount of flocs |

**Table 6. pH measurement results of formulation formulas in accelerated stability tests at 25 °C or 40 °C**

| Formula No. | T0 | 25°C | | 40°C | | |
|---|---|---|---|---|---|---|
| | | 2W | 4W | 1W | 2W | 4W |
| F1 | 4.6 | 4.6 | 4.6 | 4.6 | 4.7 | 4.5 |
| F2 | 5.0 | 5.1 | 5.1 | 5.1 | 5.1 | 5.0 |
| F3 | 5.5 | 5.5 | 5.5 | 5.5 | 5.5 | 5.4 |
| F4 | 5.1 | 5.0 | 5.1 | 5.0 | 5.1 | 5.0 |
| F5 | 5.5 | 5.5 | 5.5 | 5.5 | 5.5 | 5.5 |
| F6 | 5.9 | 5.9 | 5.9 | 5.8 | 6.0 | 6.0 |
| F7 | 5.6 | 5.6 | 5.5 | 5.5 | 5.6 | 5.5 |
| F8 | 6.1 | 6.1 | 6.0 | 5.9 | 6.1 | 6.0 |
| F9 | 6.6 | 6.6 | 6.6 | 6.4 | 6.6 | 6.5 |
| F10 | 6.4 | 6.5 | 6.5 | 6.4 | 6.4 | 6.4 |
| F11 | 6.9 | 6.9 | 7.0 | 6.9 | 7.0 | 6.9 |
| F12 | 7.4 | 7.5 | 7.5 | 7.4 | 7.4 | 7.4 |

**Table 7. Concentration (mg/mL) measurement results of formulation formulas in accelerated stability tests at 25 °C or 40 °C**

| Formula No. | T0 | 25°C | | 40°C | | |
|---|---|---|---|---|---|---|
| | | 2W | 4W | 1W | 2W | 4W |
| F1 | 1.95 | 1.95 | 2.00 | 1.96 | 1.96 | 1.93 |
| F2 | 1.93 | 1.95 | 1.93 | 1.98 | 1.93 | 1.99 |
| F3 | 1.91 | 1.89 | 1.92 | 1.88 | 1.89 | 1.90 |
| F4 | 1.97 | 1.94 | 1.96 | 1.83 | 1.74 | 1.43 |
| F5 | 1.88 | 1.86 | 1.85 | 1.83 | 1.81 | 1.74 |
| F6 | 1.95 | 1.91 | 1.96 | 1.91 | 1.92 | 1.96 |
| F7 | 1.91 | 1.89 | 1.99 | 1.88 | 1.87 | 1.91 |
| F8 | 1.87 | 1.82 | 1.93 | 1.86 | 1.84 | 1.84 |
| F9 | 2.01 | 1.96 | 1.96 | 1.94 | 1.97 | 1.95 |
| F10 | 1.67 | 1.61 | 1.62 | 1.6 | 1.59 | 1.63 |
| F11 | 1.89 | 1.84 | 1.87 | 1.82 | 1.84 | 1.88 |
| F12 | 1.87 | 1.83 | 1.84 | 1.82 | 1.84 | 1.87 |

**Table 8. DLS (radius, nm) measurement results of formulation formulas in accelerated stability tests at 25 °C or 40 °C**

| Formula No. | T0 | 25°C | | 40°C | | |
|---|---|---|---|---|---|---|
| | | 2W | 4W | 1W | 2W | 4W |
| F1 | 6 | 6 | 5 | 6 | 6 | 6 |
| F2 | 6 | 8 | 6 | 21 | 24 | 28 |
| F3 | 6 | 7 | 5 | 6 | 7 | 12 |
| F4 | 6 | 6 | 5 | 7 | 9 | 8 |
| F5 | 6 | 6 | 6 | 6 | 6 | 6 |
| F6 | 6 | 6 | 6 | 6 | 6 | 7 |
| F7 | 5 | 6 | 7 | 6 | 6 | 7 |
| F8 | 6 | 7 | 5 | 6 | 6 | 7 |
| F9 | 6 | 6 | 6 | 6 | 5 | 6 |
| F10 | 6 | 6 | 9 | 6 | 7 | 10 |
| F11 | 6 | 6 | 6 | 7 | 8 | 11 |
| F12 | 7 | 6 | 6 | 7 | 8 | 8 |

**Table 9. SEC-HPLC detection results of formulation formulas in accelerated stability tests at 25 °C**

| Formula No. | T0 | | | 25 °C | | | | | |
|---|---|---|---|---|---|---|---|---|---|
| | | | | 2W | | | 4W | | |
| | Main peak (%) | Polymer (%) | Oligomer (%) | Main peak (%) | Polymer (%) | Oligomer (%) | Main peak (%) | Polymer (%) | Oligomer (%) |
| F1 | 98.75 | 1.25 | ND | 97.91 | 1.95 | 0.15 | 98.89 | 0.87 | 0.24 |
| F2 | 99.35 | 0.65 | ND | 99.03 | 0.97 | ND | 98.96 | 0.91 | 0.13 |
| F3 | 99.29 | 0.71 | ND | 99.15 | 0.85 | ND | 99.23 | 0.68 | 0.08 |
| F4 | 99.01 | 0.99 | ND | 98.82 | 1.03 | 0.15 | 98.84 | 0.93 | 0.23 |
| F5 | 99.01 | 0.99 | ND | 98.82 | 1.07 | 0.11 | 98.82 | 1.04 | 0.14 |
| F6 | 98.72 | 1.28 | ND | 98.58 | 1.35 | 0.07 | 98.53 | 1.35 | 0.13 |
| F7 | 99.44 | 0.56 | ND | 99.36 | 0.64 | ND | 99.15 | 0.7 | 0.15 |
| F8 | 99.55 | 0.45 | ND | 99.22 | 0.78 | ND | 99.15 | 0.74 | 0.11 |
| F9 | 99.58 | 0.42 | ND | 99.23 | 0.71 | 0.05 | 99.08 | 0.8 | 0.11 |
| F10 | 99.29 | 0.71 | ND | 98.78 | 1.16 | 0.06 | 98.52 | 1.34 | 0.14 |
| F11 | 98.59 | 1.41 | ND | 97.8 | 2.13 | 0.07 | 97.75 | 2.16 | 0.09 |
| F12 | 98.33 | 1.67 | ND | 97.4 | 2.46 | 0.15 | 96.93 | 2.79 | 0.28 |

**Table 10. SEC-HPLC detection results of formulation formulas in accelerated stability tests at 40 °C**

| Formul a No. | T0 | | | 40°C | | | | | | | | |
|---|---|---|---|---|---|---|---|---|---|---|---|---|
| | | | | 1W | | | 2W | | | 4W | | |
| | Main peak (%) | Polyme r (%) | Oligome r (%) | Main peak (%) | Polyme r (%) | Oligome r (%) | Main peak (%) | Polyme r (%) | Oligome r (%) | Main peak (%) | Polyme r (%) | Oligome r (%) |
| F1 | 98.7 5 | 1.25 | ND | 98.5 9 | 1.06 | 0.35 | 98.6 8 | 1.18 | 0.14 | 85.9 5 | 1.1 | 12.96 |
| F2 | 99.3 5 | 0.65 | ND | 99 | 0.81 | 0.19 | 98.3 3 | 1.05 | 0.61 | 98.1 4 | 1.05 | 0.8 |
| F3 | 99.2 9 | 0.71 | ND | 98.9 8 | 0.83 | 0.18 | 98.6 1 | 0.99 | 0.4 | 98.2 4 | 1.2 | 0.56 |
| F4 | 99.0 1 | 0.99 | ND | 99.4 7 | 0.15 | 0.38 | 98.7 | 1.01 | 0.29 | 95.6 5 | 0.25 | 4.1 |
| F5 | 99.0 1 | 0.99 | ND | 99.0 2 | 0.77 | 0.21 | 98.7 | 0.16 | 1.11 | 98.4 1 | 0.78 | 0.81 |
| F6 | 98.7 2 | 1.28 | ND | 98.4 1 | 1.39 | 0.2 | 98.7 4 | 0.83 | 0.43 | 97.5 6 | 1.88 | 0.56 |
| F7 | 99.4 4 | 0.56 | ND | 99.3 1 | 0.53 | 0.17 | 97.9 8 | 1.73 | 0.3 | 98.6 5 | 0.69 | 0.66 |
| F8 | 99.5 5 | 0.45 | ND | 99.0 8 | 0.76 | 0.17 | 98.8 9 | 0.74 | 0.37 | 98.4 8 | 1.09 | 0.44 |
| F9 | 99.5 8 | 0.42 | ND | 98.9 2 | 0.91 | 0.17 | 98.7 3 | 0.99 | 0.27 | 98.1 3 | 1.33 | 0.54 |
| F10 | 99.2 9 | 0.71 | ND | 98.1 8 | 1.62 | 0.2 | 98.5 3 | 1.19 | 0.27 | 94.4 7 | 4.9 | 0.63 |
| F11 | 98.5 9 | 1.41 | ND | 96.0 7 | 3.61 | 0.32 | 97.2 4 | 2.44 | 0.32 | 70.2 3 | 16.3 | 13.47 |
| F12 | 98.3 3 | 1.67 | ND | 94.1 8 | 5.26 | 0.56 | 92.6 | 6.89 | 0.51 | 53.5 6 | 20.89 | 25.55 |

**Table 11. CE-SDS (NR) detection results of formulation formulas in accelerated stability tests at 25 °C**

| Formul a No. | T0 | | | 25°C | | | | | |
|---|---|---|---|---|---|---|---|---|---|
| | | | | 2W | | | 4W | | |
| | Main peak (%) | Polymer (%) | Oligomer (%) | Main peak (%) | Polymer (%) | Oligomer (%) | Main peak (%) | Polymer (%) | Oligomer (%) |
| F1 | 99.07 | ND | 0.93 | 97.83 | ND | 2.17 | 96.21 | ND | 3.80 |
| F2 | 99.2 | ND | 0.80 | 99.25 | ND | 0.75 | 96.44 | ND | 3.55 |
| F3 | 99.34 | ND | 0.66 | 99.11 | ND | 0.89 | 96.77 | ND | 3.23 |
| F4 | 99.22 | ND | 0.78 | 99.44 | ND | 0.57 | 95.89 | ND | 4.11 |
| F5 | 98.97 | ND | 1.03 | 99.00 | ND | 1.00 | 96.05 | ND | 3.95 |
| F6 | 98.44 | ND | 1.56 | 99.16 | ND | 0.84 | 97.03 | ND | 2.96 |
| F7 | 98.22 | ND | 1.77 | 98.85 | ND | 1.15 | 92.75 | ND | 7.25 |
| F8 | 98.29 | ND | 1.71 | 98.26 | ND | 1.74 | 92.25 | ND | 7.74 |
| F9 | 98.02 | ND | 1.99 | 98.23 | ND | 1.77 | 91.76 | ND | 8.24 |
| F10 | 98.69 | ND | 1.31 | 98.05 | ND | 1.95 | 95.60 | ND | 4.40 |
| F11 | 98.97 | ND | 1.03 | 97.62 | ND | 2.39 | 94.41 | ND | 5.60 |
| F12 | 98.38 | ND | 1.62 | 97.03 | ND | 2.97 | 93.01 | ND | 6.99 |

**Table 12. CE-SDS (NR) detection results of formulation formulas in accelerated stability tests at 40 °C**

| Formula No. | T0 | | | 40°C | | | | | | | | |
|---|---|---|---|---|---|---|---|---|---|---|---|---|
| | | | | 1W | | | 2W | | | 4W | | |
| | Main peak (%) | Polymer (%) | Oligomer (%) | Main peak (%) | Polymer (%) | Oligomer (%) | Main peak (%) | Polymer (%) | Oligomer (%) | Main peak (%) | Polymer (%) | Oligomer (%) |
| F1 | 99.07 | ND | 0.93 | 99.04 | ND | 0.96 | 96.26 | ND | 3.74 | 87.82 | ND | 12.18 |
| F2 | 99.2 | ND | 0.80 | 99.31 | ND | 0.69 | 97.39 | ND | 2.61 | 89.99 | ND | 10.01 |
| F3 | 99.34 | ND | 0.66 | 99.09 | ND | 0.91 | 97.55 | ND | 2.45 | 92.29 | ND | 7.72 |
| F4 | 99.22 | ND | 0.78 | 97.16 | ND | 284 | 92.59 | ND | 7.41 | 8204 | ND | 17.95 |
| F5 | 98.97 | ND | 1.03 | 98.27 | ND | 1.73 | 96.45 | ND | 3.55 | 87.05 | ND | 12.95 |
| F6 | 98.44 | ND | 1.56 | 98.05 | ND | 1.95 | 97.84 | ND | 2.15 | 89.19 | ND | 10.81 |
| F7 | 98.22 | ND | 1.77 | 98.46 | ND | 1.53 | 97.11 | ND | 2.89 | 84.20 | ND | 15.79 |
| F8 | 98.29 | ND | 1.71 | 98.43 | ND | 1.58 | 97.38 | ND | 2.61 | 84.88 | ND | 15.12 |
| F9 | 98.02 | ND | 1.99 | 97.17 | ND | 284 | 97.6 | ND | 2.40 | 84.40 | ND | 15.60 |
| F10 | 98.69 | ND | 1.31 | 94.84 | ND | 5.16 | 92.08 | ND | 7.91 | 81.94 | ND | 1806 |
| F11 | 98.97 | ND | 1.03 | 92.08 | ND | 7.92 | 89.17 | ND | 10.83 | 73.04 | ND | 26.97 |
| F12 | 98.38 | ND | 1.62 | 87.06 | ND | 12.94 | 76.97 | ND | 23.03 | 59.99 | ND | 4002 |

**Table 13. CE-SDS (R) detection results of formulation formulas in accelerated stability tests at 25 °C**

| Formula No. | T0 | | 25 °C | | | |
|---|---|---|---|---|---|---|
| | | | 2W | | 4W | |
| | Main peak (%) | Other peaks (%) | Main peak (%) | Other peaks (%) | Main peak (%) | Other peaks (%) |
| F1 | 100 | ND | 98.67 | 1.34 | 96.32 | 3.68 |
| F2 | 100 | ND | 98.8 | 1.21 | 98.46 | 1.55 |
| F3 | 100 | ND | 97.67 | 2.33 | 97.85 | 2.14 |
| F4 | 100 | ND | 98.45 | 1.55 | 96.63 | 3.39 |
| F5 | 100 | ND | 98.94 | 1.06 | 97.42 | 2.58 |
| F6 | 100 | ND | 98.84 | 1.16 | 97.2 | 2.8 |
| F7 | 100 | ND | 98.39 | 1.61 | 97.72 | 2.29 |
| F8 | 100 | ND | 98.86 | 1.16 | 98.12 | 1.89 |
| F9 | 100 | ND | 98.77 | 1.23 | 98.05 | 1.95 |
| F10 | 100 | ND | 99.02 | 0.98 | 96.8 | 3.21 |
| F11 | 100 | ND | 98.61 | 1.39 | 97.03 | 2.95 |
| F12 | 100 | ND | 98.05 | 1.94 | 95.98 | 4.02 |

**Table 14. CE-SDS (R) detection results of formulation formulas in accelerated stability tests at 40 °C**

| Formul a No. | T0 | | 40°C | | | | | |
|---|---|---|---|---|---|---|---|---|
| | | | 1W | | 2W | | 4W | |
| | Main peak (%) | Other peaks (%) | Main peak (%) | Other peaks (%) | Main peak (%) | Other peaks (%) | Main peak (%) | Other peaks (%) |
| F1 | 100 | ND | 90.26 | 9.74 | 87.39 | 12.6 | 79.63 | 20.37 |
| F2 | 100 | ND | 93.55 | 6.45 | 92.87 | 7.14 | 88.36 | 11.64 |
| F3 | 100 | ND | 94.79 | 5.23 | 95.95 | 4.05 | 94.75 | 5.25 |
| F4 | 100 | ND | 82.34 | 17.68 | 75.79 | 24.22 | 66.64 | 33.38 |
| F5 | 100 | ND | 91.82 | 8.18 | 88.79 | 11.2 | 81.64 | 18.34 |
| F6 | 100 | ND | 95.68 | 4.33 | 95.83 | 4.18 | 94.27 | 5.73 |
| F7 | 100 | ND | 93.91 | 6.09 | 94.51 | 5.48 | 87.8 | 12.2 |
| F8 | 100 | ND | 96.21 | 3.79 | 98.82 | 1.18 | 96.07 | 3.92 |
| F9 | 100 | ND | 97.03 | 2.97 | 98.54 | 1.45 | 96.42 | 3.59 |
| F10 | 100 | ND | 95.43 | 4.58 | 93.78 | 6.22 | 84.78 | 15.22 |
| F11 | 100 | ND | 90.72 | 9.29 | 87.88 | 12.11 | 77.94 | 22.05 |
| F12 | 100 | ND | 87.27 | 12.72 | 80.89 | 19.12 | 65.49 | 34.5 |

### 3. Investigation results

(1) Appearance ranking: F1 and F3 > F2, F7, F8, F9, and F11 > F10 and F12 > F4, F5, and F6.
(2) pH value: the pH values of the 12 buffer systems were stable and had no significant change.
(3) Protein concentration ranking: F1, F2, F3, F6, F7, F8, F9, F11, and F12 > F5 > F4 and F10.
(4) DLS ranking: F1, F5, and F9 > F6, F7, and F8 > F3, F4, F10, F11, and F12 > F2.
(5) SEC-HPLC ranking: F2, F3, F5, F6, F7, F8, and F9 > F4 and F10 > F1 > F11 and F12.
(6) CE-SDS (NR) ranking: F2, F3, and F6 >F1, F5, F7, F8, and F9 > F4 and F10 > F11 and F12.
(7) CE-SDS (R) ranking: F8 and F9 > F3 and F6 > F2, F7, and F10 > F1, F4, F5, F11, and F12.

Summary: by comprehensively considering the indexes detected in the accelerated stability tests at 25 °C and the accelerated stability tests at 40 °C, formula F3 (20 mM acetic acid-sodium acetate, pH 5.5) was superior to other formulas and showed better stability. Therefore, F3 was selected for the next development.

### Example 3. Excipient Screening

### 1. Screening protocol

On the basis of the optimal buffer system (20 mM acetic acid-sodium acetate, pH 5.5) screened in Example 2, 7 formulas were designed. The stability of the IL2 mutant fusion protein (the concentration of the IL2 mutant fusion protein was 2 mg/mL) was independently investigated after the addition of sodium chloride, glycine, arginine hydrochloride, sucrose, trehalose, mannitol, or sorbitol, and 1 optimal formulation formula was selected for subsequent surfactant screening. The investigation indexes include: appearance, pH value, concentration, SEC-HPLC, DLS, and CE-SDS (NR & R). The specific formulation and investigation protocol are shown in Table 15.

**Table 15. Excipient screening protocol**

| Formula | Formulation | | T0 | 2-8°C | 25°C | | 40°C | | |
|---|---|---|---|---|---|---|---|---|---|
| | | | | 4W | 2W | 4W | 1W | 2W | 4W |
| | Buffer system, pH | Excipient | | | | | | | |
| F3-1 | 20 mM acetic acid-sodium acetate, pH 5.5 | 2% (w/v) glycine | X | X | X | X | X | X | X |
| F3-2 | | 140 mM arginine hydrochloride | X | X | X | X | X | X | X |
| F3-3 | | 8% (w/v) sucrose | X | X | X | X | X | X | X |
| F3-4 | | 8% (w/v) trehalose | X | X | X | X | X | X | X |
| F3-5 | | 4.5% (w/v) mannitol | X | X | X | X | X | X | X |
| F3-6 | | 4.5% (w/v) sorbitol | X | X | X | X | X | X | X |
| F3-7 | | 140 mM sodium chloride | X | X | X | X | X | X | X |

| | | | | | | | | | |
|---|---|---|---|---|---|---|---|---|---|
| Note: X = appearance, pH value, concentration, DLS, SEC-HPLC, and CE-SDS (NR & R). | | | | | | | | | |

### 2. Excipient screening results

The major results of the excipient screening are summarized below.

**Table 16. Appearance detection results of formulation formulas at different temperatures/time points**

| Formul a | T0 | 2-8°C | 25°C | | 40°C | | |
|---|---|---|---|---|---|---|---|
| | | 4W | 2W | 4W | 1W | 2W | 4W |
| F3-1 | Colorless, clear | Colorless, clear | Colorless, clear | Colorless, with fine ciliated insoluble matter | Colorless, with fine ciliated insoluble matter | Colorless, with fine ciliated insoluble matter | Colorless, with flocs |
| F3-2 | Colorless, clear | Colorless, clear | Colorless, clear | Colorless, clear | Colorless, with fine ciliated insoluble matter | Colorless, with fine ciliated insoluble matter | Colorless, with fine ciliated insoluble matter |
| F3-3 | Colorless, clear | Colorless, clear | Colorless, with fine ciliated insoluble matter | Colorless, with fine ciliated insoluble matter | Colorless, with more fine ciliated insoluble matter | Colorless, with flocs | Colorless, with flocs |
| F3-4 | Colorless, clear | Colorless, with fine ciliated insoluble | Colorless, with a small amount of fine ciliated | Colorless, with fine ciliated insoluble | Colorless, with a small amount of fine ciliated | Colorless, with fine ciliated insoluble | Colorless, with flocs |
| | | matter | insoluble matter | matter | insoluble matter | matter | |
| F3-5 | Colorless, clear | Colorless, with fine ciliated insoluble matter | Colorless, clear | Colorless, with fine ciliated insoluble matter | Colorless, with flocs | Colorless, with flocs | Colorless, with flocs |
| F3-6 | Colorless, clear | Colorless, with fine ciliated insoluble matter | Colorless, with a small amount of fine ciliated insoluble matter | Colorless, with fine ciliated insoluble matter | Colorless, with fine ciliated insoluble matter | Colorless, with fine ciliated insoluble matter | Colorless, with flocs |
| F3-7 | Colorless, clear | Colorless, with fine ciliated insoluble matter | Colorless, with fine ciliated insoluble matter | Colorless, with fine ciliated insoluble matter | Colorless, with more long ciliated insoluble matter | Colorless, with more flocs | Colorless, with more flocs |

**Table 17. pH value or protein concentration measurement results of formulation formulas at different temperatures/time points**

| Formul a No. | Measurement index: pH | | | | | | | Measurement index: protein concentration (mg/mL) | | | | | | |
|---|---|---|---|---|---|---|---|---|---|---|---|---|---|---|
| | T0 | 2-8°C | 25°C | | 40°C | | | T0 | 2-8°C | 25°C | | 40°C | | |
| | | 4W | 2W | 4W | 1W | 2W | 4W | | 4W | 2W | 4W | 1W | 2W | 4W |
| F3-1 | 5.5 | 5.6 | 5.5 | 5.5 | 5.6 | 5.5 | 5.6 | 1.92 | 1.92 | 1.92 | 1.93 | 1.90 | 1.92 | 1.94 |
| F3-2 | 5.4 | 5.4 | 5.3 | 5.4 | 5.4 | 5.3 | 5.4 | 2.03 | 2.04 | 2.03 | 2.03 | 1.99 | 1.99 | 2.02 |
| F3-3 | 5.5 | 5.5 | 5.5 | 5.6 | 5.6 | 5.5 | 5.6 | 1.99 | 2.03 | 2.02 | 2.05 | 1.98 | 2.00 | 2.03 |
| F3-4 | 5.5 | 5.5 | 5.5 | 5.5 | 5.5 | 5.5 | 5.5 | 1.98 | 2.03 | 1.99 | 2.01 | 1.98 | 1.99 | 2.02 |
| F3-5 | 5.5 | 5.5 | 5.4 | 5.5 | 5.5 | 5.5 | 5.5 | 1.95 | 1.98 | 1.96 | 2.00 | 1.94 | 1.96 | 1.98 |
| F3-6 | 5.5 | 5.5 | 5.4 | 5.5 | 5.5 | 5.4 | 5.5 | 1.95 | 1.96 | 1.98 | 2.00 | 1.94 | 1.96 | 1.98 |
| F3-7 | 5.4 | 5.4 | 5.4 | 5.4 | 5.4 | 5.4 | 5.5 | 1.97 | 1.98 | 1.98 | 1.97 | 1.92 | 1.94 | 1.95 |

**Table 18. DLS (diameter, nm) measurement results of formulation formulas at different temperatures/time points**

| Formula No. | T0 | 2-8°C | 25°C | | 40°C | | |
|---|---|---|---|---|---|---|---|
| | | 4W | 2W | 4W | 1W | 2W | 4W |
| F3-1 | 10 | 10 | 12 | 10 | 11 | 11 | 13 |
| F3-2 | 11 | 10 | 10 | 11 | 11 | 11 | 11 |
| F3-3 | 11 | 13 | 16 | 11 | 13 | 18 | 15 |
| F3-4 | 12 | 13 | 17 | 12 | 18 | 17 | 18 |
| F3-5 | 11 | 13 | 12 | 10 | 11 | 15 | 17 |
| F3-6 | 11 | 12 | 13 | 11 | 15 | 19 | 16 |
| F3-7 | 29 | 16 | 19 | 17 | 24 | 26 | 27 |

**Table 19-1. SEC-HPLC detection results of formulation formulas at different temperatures/time points**

| Formal a No. | T0 | | | 2-8°C | | | 25°C | | | | | |
|---|---|---|---|---|---|---|---|---|---|---|---|---|
| | | | | 4W | | | 2W | | | 4W | | |
| | Main peak (%) | Polyme r (%) | Oligome r (%) | Main peak (%) | Polyme r (%) | Oligome r (%) | Mai n peak (%) | Polyme r (%) | Oligome r (%) | Mai n peak (%) | Polyme r (%) | Oligome r (%) |
| F3-1 | 99.9 | 0.1 | ND | 99.4 | 0.6 | ND | 100 | ND | 0 | 99.2 | 0.7 | 0 |
| F3-2 | 99.6 | 0.4 | ND | 98.9 | 1.1 | ND | 99.7 | 0.2 | 0 | 98.8 | 1.1 | 0.1 |
| F3-3 | 99.9 | 0.1 | ND | 99.4 | 0.6 | ND | 99.9 | 0.1 | 0 | 99.3 | 0.7 | 0 |
| F3-4 | 99.9 | 0.1 | ND | 99.4 | 0.6 | ND | 99.9 | 0.1 | 0 | 99.1 | 0.8 | 0 |
| F3-5 | 99.9 | 0.1 | ND | 99.3 | 0.7 | ND | 99.9 | 0.1 | 0 | 99.2 | 0.8 | 0.1 |
| F3-6 | 99.9 | 0.1 | ND | 99.3 | 0.7 | ND | 99.9 | 0.1 | 0 | 99.2 | 0.7 | 0 |
| F3-7 | 99.7 | 0.3 | ND | 99.1 | 0.9 | ND | 99.8 | 0.2 | 0 | 98.9 | 1 | 0 |

**Table 19-2. SEC-HPLC detection results of formulation formulas at different temperatures/time points**

| Formula No. | T0 | | | 40°C | | | | | | | | |
|---|---|---|---|---|---|---|---|---|---|---|---|---|
| | | | | 1W | | | 2W | | | 4W | | |
| | Main peak (%) | Polymer (%) | Oligomer (%) | Main peak (%) | Polymer (%) | Oligomer (%) | Main peak (%) | Polym er (%) | Oligomer (%) | Main peak (%) | Polymer (%) | Oligomer (%) |
| F3-1 | 99.9 | 0.1 | ND | 99.2 | 0.5 | 0.3 | 99.3 | 0.1 | 0.6 | 98.4 | 1 | 0.6 |
| F3-2 | 99.6 | 0.4 | ND | 99.0 | 0.5 | 0.5 | 99.2 | 0.1 | 0.8 | 97.5 | 0.9 | 1.7 |
| F3-3 | 99.9 | 0.1 | ND | 99.7 | 0.1 | 0.2 | 99.2 | 0.2 | 0.5 | 98.3 | 1.1 | 0.6 |
| F3-4 | 99.9 | 0.1 | ND | 99.8 | 0 | 0.2 | 99.3 | 0.2 | 0.5 | 98.2 | 1.2 | 0.6 |
| F3-5 | 99.9 | 0.1 | ND | 99.7 | 0 | 0.2 | 99.2 | 0.2 | 0.6 | 98.3 | 1.1 | 0.6 |
| F3-6 | 99.9 | 0.1 | ND | 99.7 | 0 | 0.3 | 99.2 | 0.3 | 0.5 | 98.2 | 1.2 | 0.6 |
| F3-7 | 99.7 | 0.3 | ND | 99.8 | 0 | 0.2 | 99.3 | 0.1 | 0.6 | 97.9 | 0.8 | 1.3 |

**Table 20-1. CE-SDS (R) detection results of formulation formulas at different temperatures/time points**

| Formula No. | T0 | | 2~8°C | | 25°C | | | |
|---|---|---|---|---|---|---|---|---|
| | | | 4W | | 2W | | 4W | |
| | Main peak (%) | Other peaks (%) | Main peak (%) | Other peaks (%) | Main peak (%) | Other peaks (%) | Main peak (%) | Other peaks (%) |
| F3-1 | 99.45 | 0.55 | 99.00 | 1.00 | 99.30 | 0.71 | 98.52 | 1.48 |
| F3-2 | 99.22 | 0.78 | 99.32 | 0.67 | 98.87 | 1.14 | 98.12 | 1.89 |
| F3-3 | 99.41 | 0.59 | 99.34 | 0.66 | 98.68 | 1.32 | 98.83 | 1.16 |
| F3-4 | 99.45 | 0.55 | 99.39 | 0.60 | 99.10 | 0.90 | 98.77 | 1.23 |
| F3-5 | 98.92 | 1.08 | 99.42 | 0.59 | 99.23 | 0.77 | 98.80 | 1.21 |
| F3-6 | 99.45 | 0.55 | 99.42 | 0.57 | 99.09 | 0.90 | 98.93 | 1.07 |
| F3-7 | 99.39 | 0.61 | 99.41 | 0.59 | 98.35 | 1.64 | 98.37 | 1.62 |

**Table 20-2. CE-SDS (R) detection results of formulation formulas at different temperatures/time points**

| Formula No. | T0 | | 40°C | | | | | |
|---|---|---|---|---|---|---|---|---|
| | | | 1W | | 2W | | 4W | |
| | Main peak (%) | Other peaks (%) | Main peak (%) | Other peaks (%) | Main peak (%) | Other peaks (%) | Main peak (%) | Other peaks (%) |
| F3-1 | 99.45 | 0.55 | 98.13 | 1.86 | 96.75 | 3.24 | 95.35 | 4.64 |
| F3-2 | 99.22 | 0.78 | 95.31 | 4.67 | 90.09 | 9.90 | 85.42 | 14.58 |
| F3-3 | 99.41 | 0.59 | 98.20 | 1.81 | 97.21 | 2.80 | 95.69 | 4.30 |
| F3-4 | 99.45 | 0.55 | 98.22 | 1.79 | 97.02 | 2.99 | 95.93 | 4.08 |
| F3-5 | 98.92 | 1.08 | 97.91 | 2.10 | 96.85 | 3.15 | 95.80 | 4.20 |
| F3-6 | 99.45 | 0.55 | 98.00 | 2.00 | 96.84 | 3.16 | 95.78 | 4.21 |
| F3-7 | 99.39 | 0.61 | 96.34 | 3.64 | 93.12 | 6.89 | 90.15 | 9.85 |

**Table 21-1. CE-SDS (NR) detection results of formulation formulas at different temperatures/time points**

| Formula No. | T0 | | | 2~8°C | | | 25°C | | | | | |
|---|---|---|---|---|---|---|---|---|---|---|---|---|
| | | | | 4W | | | 2W | | | 4W | | |
| | Main peak (%) | Polymer (%) | Oligomer (%) | Main peak (%) | Polymer (%) | Oligomer (%) | Main peak (%) | Polymer (%) | Oligomer (%) | Main peak (%) | Polymer (%) | Oligomer (%) |
| F3-1 | 98.55 | 0 | 1.45 | 98.45 | 0.08 | 1.47 | 98.34 | 0.10 | 1.56 | 97.97 | 0.15 | 1.88 |
| F3-2 | 98.62 | 0 | 1.38 | 98.51 | 0.06 | 1.43 | 98.23 | 0.06 | 1.71 | 98.23 | 0.08 | 1.68 |
| F3-3 | 98.51 | 0 | 1.49 | 98.47 | 0.08 | 1.46 | 98.34 | 0.10 | 1.57 | 97.99 | 0.16 | 1.85 |
| F3-4 | 98.51 | 0 | 1.49 | 98.46 | 0.08 | 1.46 | 98.34 | 0.10 | 1.57 | 98.17 | 0.12 | 1.70 |
| F3-5 | 98.55 | 0 | 1.45 | 98.54 | 0.10 | 1.36 | 98.28 | 0.07 | 1.64 | 98.14 | 0.10 | 1.76 |
| F3-6 | 98.63 | 0 | 1.37 | 98.38 | 0.09 | 1.53 | 98.17 | 0.06 | 1.77 | 97.98 | 0.11 | 1.92 |
| F3-7 | 98.58 | 0 | 1.42 | 98.17 | 0.06 | 1.77 | 98.25 | 0.10 | 1.64 | 97.91 | 0.14 | 1.95 |

**Table 21-2. CE-SDS (NR) detection results of formulation formulas at different temperatures/time points**

| Formula | T0 | | | 40°C | | | | | | | | |
|---|---|---|---|---|---|---|---|---|---|---|---|---|
| No. | | | | 1W | | | 2W | | | 4W | | |
| | Main peak (%) | Polymer (%) | Oligomer (%) | Main peak (%) | Polymer (%) | Oligomer (%) | Main peak (%) | Polymer (%) | Oligomer (%) | Main peak (%) | Polymer (%) | Oligomer (%) |
| F3-1 | 98.55 | 0 | 1.45 | 97.26 | 0.12 | 2.62 | 96.00 | 0.12 | 3.88 | 92.91 | 0.22 | 6.87 |
| F3-2 | 98.62 | 0 | 1.38 | 97.89 | 0.15 | 1.96 | 97.20 | 0.14 | 2.66 | 95.19 | 0.14 | 4.67 |
| F3-3 | 98.51 | 0 | 1.49 | 97.63 | 0.18 | 2.19 | 97.16 | 0.11 | 2.73 | 95.31 | 0.16 | 4.54 |
| F3-4 | 98.51 | 0 | 1.49 | 97.82 | 0.14 | 2.05 | 97.13 | 0.15 | 2.71 | 94.34 | 0.08 | 5.58 |
| F3-5 | 98.55 | 0 | 1.45 | 97.80 | 0.11 | 2.10 | 96.93 | 0.12 | 2.95 | 94.25 | 0.16 | 5.59 |
| F3-6 | 98.63 | 0 | 1.37 | 97.23 | 0.08 | 2.69 | 95.52 | 0.10 | 4.38 | 93.01 | 0.11 | 6.88 |
| F3-7 | 98.58 | 0 | 1.42 | 97.58 | 0.16 | 2.26 | 96.74 | 0.18 | 3.08 | 94.79 | 0.22 | 4.99 |

### 3. Investigation results

(1) Appearance ranking: F3-2 > F3-1 > F3-3 > F3-5> F3-4 and F3-6 > F3-7.
(2) pH value ranking: the data showed no significant differences among the groups.
(3) Concentration ranking: the data showed no significant differences among the groups.
(4) DLS ranking: F3-2 > F3-1 > F3-3, F3-4, F3-5, and F3-6 > F3-7.
(5) SEC-HPLC ranking: F3-1, F3-3, F3-4, F3-5, and F3-6 > F3-7 > F3-2.
(6) CE-SDS (R) ranking: F3-1, F3-3, F3-4, F3-5, and F3-6 > F3-7 > F3-2.
(7) CE-SDS (NR) ranking: F3-2 and F3-3 > F3-4 > F3-5 > F3-7 > F3-6 > F3-1.

**Summary:** according to the appearance, the groups, F3-4, F3-5, F3-6, and F3-7, with the presence of particles in the long-term stability tests at 2-8 °C, were firstly excluded. Formula F3-1 containing glycine had higher oligomer content than those of F3-2 and F3-3 at 40 °C for 4 W, as detected by CE-SDS (NR), indicating that the IL-2 protein is more easily degraded in formula F3-1. For F3-2, the SEC-HPLC detection index was not greatly different from other groups, and the proportion of other peaks in CE-SDS (R) was higher at 40 °C for 4 W, but there was no significant difference from other groups in the long-term stability tests at 2-8 °C and 25 °C. Therefore, formula F3-2 and formula F3-3 were superior to other formulas and showed good stability by comprehensively considering the stability investigation data. Formula F3-2 (containing 140 mM arginine hydrochloride) and formula F3-3 (containing 8% w/v sucrose) were selected for further investigation of the stability of the formulas after the addition of a surfactant.

### Example 4. Surfactant Investigation

### 1. Investigation protocol

On the basis of formula F3-2 and formula F3-3, the stability of the formulas after the addition of the surfactant PS-80 was investigated. The formulas are detailed in Table 22. The investigation protocol is detailed in Table 23. The investigation indexes include appearance, pH value, protein concentration, DLS, SEC-HPLC, CE-SDS (NR & R), and insoluble particle (MFI). According to the investigation results, a proper formulation formula was selected to confirm the stability of the formula.

**Table 22. Formulation formulas**

| Formula No. | Formulation | | Concentration of IL2 mutant fusion protein |
|---|---|---|---|
| F3-2-1 | 20 mM acetic acid-sodium acetate, 140 mM arginine hydrochloride, pH 5.5 | 0.04% (w/v) polysorbate 80 | 2 mg/mL |
| F3-2-2 | | 0.06% (w/v) polysorbate 80 | 2 mg/mL |
| F3-2-3 | | 0.04% (w/v) polysorbate 80 | 5 mg/mL |
| F3-2-4 | | 0.06% (w/v) polysorbate 80 | 5 mg/mL |
| F3-3-1 | 20 mM acetic acid-sodium acetate, 8% (w/v) sucrose, pH 5.5 | 0.04% (w/v) polysorbate 80 | 2mg/mL |

**Table 23. Investigation protocol**

| Investigation condition | T0 | Sampling point | | | |
|---|---|---|---|---|---|
| 2~8°C | X | 4W | | 3M | |
| | | X | | X | |
| 25 °C | X | 4W | | 3M | |
| | | X | | X | |
| 40°C | X | 1W | 2W | | 4W |
| | | X | | X | |
| Shaking test (300 rpm, 25 °C) | X | 4D | | 7D | |
| | | X | | X | |
| Freezing-thawing test (-80 °C to 25 °C) | X | 3C | | 5C | |
| | | X | | X | |

| | | | | | |
|---|---|---|---|---|---|
| Note: X = appearance, pH value, protein concentration, DLS, SEC, CE-SDS (NR & R), and insoluble particle (MFI). | | | | | |

Shaking test (300 rpm, 25 °C): 25 °C, 300 rpm, continuous shaking.

Freezing-thawing test (-80 °C to 25 °C): multiple cycles of freezing-thawing were performed. In each cycle of freezing-thawing, the samples were placed at -80 °C until frozen, and then placed at 25 °C until completely thawed, forming 1 cycle of freezing-thawing.

### 2. Screening results

The major results are summarized below:

**Table 24. Appearance detection results at different temperatures/time points**

| Condition | Time point | F3-2-1 | F3-2-2 | F3-2-3 | F3-2-4 | F3-3-1 |
|---|---|---|---|---|---|---|
| 2-8°C | T0 | Colorless, clear, no visible particles | Colorless, clear, no visible particles | Colorless, clear, no visible particles | Colorless, clear, no visible particles | Colorless, clear, no visible particles |
| | 4W | Colorless, clear, no visible particles | Colorless, clear, no visible particles | Colorless, clear, no visible particles | Colorless, clear, no visible particles | Colorless, clear, no visible particles |
| | 3M | Colorless, clear, no visible particles | Colorless, clear, no visible particles | Colorless, clear, no visible particles | Colorless, clear, no visible particles | Colorless, clear, no visible particles |
| 25 °C | T0 | Colorless, clear, no visible particles | Colorless, clear, no visible particles | Colorless, clear, no visible particles | Colorless, clear, no visible particles | Colorless, clear, no visible particles |
| | 4W | Colorless, clear, no visible particles | Colorless, clear, no visible particles | Colorless, clear, no visible particles | Colorless, clear, no visible particles | Colorless, clear, no visible particles |
| | 3M | Colorless, clear, no visible particles | Colorless, clear, no visible particles | Colorless, clear, no visible particles | Colorless, clear, no visible particles | Colorless, clear, no visible particles |
| 40°C | T0 | Colorless, clear, no visible particles | Colorless, clear, no visible particles | Colorless, clear, no visible particles | Colorless, clear, no visible particles | Colorless, clear, no visible particles |
| | 1W | Colorless, clear, no visible particles | Colorless, clear, no visible particles | Colorless, clear, no visible particles | Colorless, clear, no visible particles | Colorless, clear, no visible particles |
| | 2W | Colorless, clear, no visible particles | Colorless, clear, no visible particles | Colorless, clear, no visible particles | Colorless, clear, no visible particles | Colorless, clear, no visible particles |
| | 4W | Colorless, clear, no visible particles | Colorless, clear, no visible particles | Colorless, clear, no visible particles | Colorless, clear, no visible particles | Colorless, clear, no visible particles |
| Shaking test | T0 | Colorless, clear, no visible particles | Colorless, clear, no visible particles | Colorless, clear, no visible particles | Colorless, clear, no visible particles | Colorless, clear, no visible particles |
| | 4D | Colorless, clear, no visible particles | Colorless, clear, no visible particles | Colorless, clear, no visible particles | Colorless, clear, no visible particles | Colorless, clear, no visible particles |
| | 7D | Colorless, clear, no visible particles | Colorless, clear, no visible particles | Colorless, clear, no visible particles | Colorless, clear, no visible particles | Colorless, clear, no visible particles |
| Freezing-thawin g test | T0 | Colorless, clear, no visible particles | Colorless, clear, no visible particles | Colorless, clear, no visible particles | Colorless, clear, no visible particles | Colorless, clear, no visible particles |
| | 3C | Colorless, clear, no visible particles | Colorless, clear, no visible particles | Colorless, clear, no visible particles | Colorless, clear, no visible particles | Colorless, clear, no visible particles |
| | 5C | Colorless, clear, no visible particles | Colorless, clear, no visible particles | Colorless, clear, no visible particles | Colorless, clear, no visible particles | Colorless, clear, no visible particles |

**Table 25. pH measurement results at different temperatures/time points**

| Condition | Time point | F3-2-1 | F3-2-2 | F3-2-3 | F3-2-4 | F3-3-1 |
|---|---|---|---|---|---|---|
| 2-8°C | T0 | 5.4 | 5.4 | 5.4 | 5.4 | 5.5 |
| | 4W | 5.4 | 5.4 | 5.4 | 5.4 | 5.5 |
| | 3M | 5.4 | 5.4 | 5.4 | 5.4 | 5.6 |
| 25°C | T0 | 5.4 | 5.4 | 5.4 | 5.4 | 5.5 |
| | 4W | 5.4 | 5.4 | 5.4 | 5.4 | 5.5 |
| | 3M | 5.4 | 5.4 | 5.4 | 5.4 | 5.6 |
| 40°C | T0 | 5.4 | 5.4 | 5.4 | 5.4 | 5.5 |
| | 1W | 5.4 | 5.4 | 5.4 | 5.4 | 5.5 |
| | 2W | 5.4 | 5.4 | 5.4 | 5.4 | 5.5 |
| | 4W | 5.4 | 5.4 | 5.5 | 5.5 | 5.6 |
| Shaking test | T0 | 5.4 | 5.4 | 5.4 | 5.4 | 5.5 |
| | 4D | 5.4 | 5.4 | 5.4 | 5.4 | 5.5 |
| | 7D | 5.4 | 5.4 | 5.4 | 5.4 | 5.5 |
| Freezing-thawing test | T0 | 5.4 | 5.4 | 5.4 | 5.4 | 5.5 |
| | 3C | 5.4 | 5.4 | 5.4 | 5.4 | 5.5 |
| | 5C | 5.4 | 5.4 | 5.4 | 5.4 | 5.5 |

**Table 26. Protein concentration (mg/mL) measurement results at different temperatures/time points**

| Condition | Time point | F3-2-1 | F3-2-2 | F3-2-3 | F3-2-4 | F3-3-1 |
|---|---|---|---|---|---|---|
| 2-8°C | T0 | 2.03 | 2.07 | 4.96 | 4.97 | 1.99 |
| | 4W | 2.05 | 2.04 | 4.97 | 4.98 | 2.00 |
| | 3M | 2.03 | 2.03 | 4.97 | 4.98 | 1.98 |
| 25°C | T0 | 2.03 | 2.07 | 4.96 | 4.97 | 1.99 |
| | 4W | 2.07 | 2.05 | 4.98 | 4.97 | 2.01 |
| | 3M | 2.03 | 2.03 | 4.96 | 4.98 | 1.98 |
| 40°C | T0 | 2.03 | 2.07 | 4.96 | 4.97 | 1.99 |
| | 1W | 2.04 | 2.06 | 4.98 | 4.97 | 2.00 |
| | 2W | 2.05 | 2.04 | 4.95 | 4.98 | 2.00 |
| | 4W | 2.04 | 2.04 | 4.95 | 4.96 | 1.99 |
| Shaking test | T0 | 2.03 | 2.07 | 4.96 | 4.97 | 1.99 |
| | 4D | 2.06 | 2.05 | 4.99 | 4.99 | 2.01 |
| | 7D | 2.04 | 2.05 | 4.94 | 4.99 | 1.99 |
| Freezing-thawing test | T0 | 2.03 | 2.07 | 4.96 | 4.97 | 1.99 |
| | 3C | 2.04 | 2.02 | 4.97 | 5.01 | 2.00 |
| | 5C | 2.02 | 2.04 | 4.98 | 4.97 | 1.99 |

**Table 27. DLS (diameter, nm) measurement results at different temperatures/time points**

| Condition | Time point | F3-2-1 | F3-2-2 | F3-2-3 | F3-2-4 | F3-3-1 |
|---|---|---|---|---|---|---|
| 2-8°C | T0 | 9 | 10 | 10 | 10 | 10 |
| | 4W | 9 | 10 | 9 | 9 | 9 |
| | 3M | 10 | 10 | 10 | 9 | 11 |
| 25 °C | T0 | 9 | 10 | 10 | 10 | 10 |
| | 4W | 9 | 9 | 10 | 10 | 9 |
| | 3M | 9 | 9 | 11 | 10 | 10 |
| 40°C | T0 | 9 | 10 | 10 | 10 | 10 |
| | 1W | 9 | 9 | 10 | 9 | 9 |
| | 2W | 10 | 10 | 10 | 10 | 10 |
| | 4W | 9 | 9 | 10 | 11 | 9 |
| Shaking test | T0 | 9 | 10 | 10 | 10 | 10 |
| | 4D | 9 | 9 | 9 | 9 | 10 |
| | 7D | 9 | 9 | 9 | 9 | 9 |
| Freezing-thawing test | T0 | 9 | 10 | 10 | 10 | 10 |
| | 3C | 9 | 9 | 9 | 9 | 10 |
| | 5C | 9 | 9 | 9 | 9 | 11 |

**Table 28. Insoluble particle (MFI, particles/mL) measurement results at different temperatures/time points**

| Condition | Time point | Particle size (µm) | F3-2-1 | F3-2-2 | F3-2-3 | F3-2-4 | F3-3-1 |
|---|---|---|---|---|---|---|---|
| 2-8°C | T0 | ≥ 10µm | 18 | 9 | 2 | 18 | 10 |
| | | ≥ 25 µm | 0 | 2 | 2 | 2 | 0 |
| | 4W | ≥ 10µm | 15 | 12 | 2 | 0 | 2 |
| | | ≥ 25 µm | 0 | 0 | 2 | 0 | 0 |
| | 3M | ≥ 10µm | 23 | 0 | 14 | 0 | 16 |
| | | ≥ 25 µm | 0 | 0 | 0 | 0 | 5 |
| 25°C | T0 | ≥ 10µm | 18 | 9 | 2 | 18 | 10 |
| | | ≥ 25 µm | 0 | 2 | 2 | 2 | 0 |
| | 4W | ≥ 10µm | 4 | 4 | 36 | 9 | 2 |
| | | ≥ 25µm | 0 | 0 | 4 | 4 | 0 |
| | 3M | ≥ 10µm | 30 | 25 | 19 | 12 | 12 |
| | | ≥ 25µm | 5 | 3 | 7 | 3 | 3 |
| 40°C | T0 | ≥ 10µm | 18 | 9 | 2 | 18 | 10 |
| | | ≥ 25µm | 0 | 2 | 2 | 2 | 0 |
| | 1W | ≥ 10µm | 22 | 13 | 7 | 38 | 28 |
| | | ≥ 25µm | 7 | 5 | 0 | 7 | 2 |
| | 2W | ≥ 10µm | 2 | 17 | 7 | 12 | 4 |
| | | ≥ 25µm | 0 | 4 | 0 | 5 | 0 |
| | 4W | ≥ 10µm | 7 | 15 | 75 | 26 | 67 |
| | | ≥ 25µm | 0 | 0 | 17 | 0 | 15 |
| Shaking test | T0 | ≥ 10µm | 18 | 9 | 2 | 18 | 10 |
| | | ≥ 25µm | 0 | 2 | 2 | 2 | 0 |
| | 4D | ≥ 10µm | 7 | 7 | 10 | 13 | 12 |
| | | ≥ 25µm | 0 | 0 | 0 | 2 | 2 |
| | 7D | ≥ 10µm | 3 | 12 | 0 | 12 | 0 |
| | | ≥ 25µm | 3 | 0 | 0 | 9 | 0 |
| Freezing-thawing test | T0 | ≥ 10µm | 18 | 9 | 2 | 18 | 10 |
| | | ≥ 25µm | 0 | 2 | 2 | 2 | 0 |
| | 3C | ≥ 10µm | 15 | 9 | 13 | 0 | 6 |
| | | ≥ 25µm | 0 | 0 | 4 | 0 | 0 |
| | 5C | ≥ 10µm | 0 | 2 | 10 | 10 | 4 |
| | | ≥ 25µm | 0 | 2 | 2 | 2 | 0 |

**Table 29. SEC-HPLC detection results at different temperatures/time points**

| Condition | Time point | SEC-HPLC | F3-2-1 | F3-2-2 | F3-2-3 | F3-2-4 | F3-3-1 |
|---|---|---|---|---|---|---|---|
| | T0 | Main peak (%) | 99.5 | 99.3 | 99.7 | 99.6 | 99.3 |
| | | Polymer (%) | 0.5 | 0.7 | 0.3 | 0.4 | 0.7 |
| | | Oligomer (%) | ND | ND | ND | ND | ND |
| | 4W | Main peak (%) | 99.5 | 99.2 | 99.7 | 99.5 | 99.5 |
| 2-8°C | | Polymer (%) | 0.5 | 0.8 | 0.3 | 0.5 | 0.5 |
| | | Oligomer (%) | ND | ND | ND | ND | ND |
| | 3M | Main peak (%) | 99.6 | 99.6 | 99.6 | 99.6 | 99.6 |
| | | Polymer (%) | 0.4 | 0.4 | 0.4 | 0.4 | 0.4 |
| | | Oligomer (%) | ND | ND | ND | ND | ND |
| | T0 | Main peak (%) | 99.5 | 99.3 | 99.7 | 99.6 | 99.3 |
| | | Polymer (%) | 0.5 | 0.7 | 0.3 | 0.4 | 0.7 |
| | | Oligomer (%) | ND | ND | ND | ND | ND |
| | 4W | Main peak (%) | 99.4 | 99.2 | 99.5 | 99.4 | 99.4 |
| 25°C | | Polymer (%) | 0.5 | 0.7 | 0.4 | 0.5 | 0.6 |
| | | Oligomer (%) | 0.1 | 0.1 | 0.1 | 0.1 | 0 |
| | 3M | Main peak (%) | 99.2 | 99.1 | 98.9 | 98.9 | 99.4 |
| | | Polymer (%) | 0.6 | 0.6 | 0.8 | 0.8 | 0.5 |
| | | Oligomer (%) | 0.3 | 0.3 | 0.3 | 0.3 | 0.1 |
| | T0 | Main peak (%) | 99.5 | 99.3 | 99.7 | 99.6 | 99.3 |
| | | Polymer (%) | 0.5 | 0.7 | 0.3 | 0.4 | 0.7 |
| | | Oligomer (%) | ND | ND | ND | ND | ND |
| | 1W | Main peak (%) | 98.9 | 98.6 | 99.2 | 99.2 | 99.5 |
| | | Polymer (%) | 0.5 | 0.7 | 0.4 | 0.4 | 0.4 |
| 40°C | | Oligomer (%) | 0.6 | 0.7 | 0.4 | 0.4 | 0.1 |
| | 2W | Main peak (%) | 98.9 | 98 | 98.6 | 98.5 | 99.7 |
| | | Polymer (%) | 0.2 | 0.5 | 0.5 | 0.6 | 0.2 |
| | | Oligomer (%) | 0.9 | 1.5 | 0.9 | 0.9 | 0.1 |
| | 4W | Main peak (%) | 98.4 | 98.1 | 98.6 | 98.4 | 98.6 |
| | | Polymer (%) | 0.7 | 0.9 | 0.6 | 0.8 | 0.8 |
| | | Oligomer (%) | 0.5 | 0.5 | 0.4 | 0.4 | 0.6 |
| | T0 | Main peak (%) | 99.5 | 99.3 | 99.7 | 99.6 | 99.3 |
| | | Polymer (%) | 0.5 | 0.7 | 0.3 | 0.4 | 0.7 |
| | | Oligomer (%) | ND | ND | ND | ND | ND |
| | 4D | Main peak (%) | 99.5 | 99.3 | 99.5 | 99.6 | 99.6 |
| Shaking test | | Polymer (%) | 0.5 | 0.6 | 0.3 | 0.4 | 0.4 |
| | | Oligomer (%) | ND | 0.1 | 0.1 | ND | ND |
| | 7D | Main peak (%) | 99.5 | 99.3 | 99.6 | 99.6 | 99.6 |
| | | Polymer (%) | 0.5 | 0.6 | 0.3 | 0.4 | 0.4 |
| | | Oligomer (%) | ND | 0.1 | 0.1 | 0 | ND |
| Freezing-thawing test | T0 | Main peak (%) | 99.5 | 99.3 | 99.7 | 99.6 | 99.3 |
| | | Polymer (%) | 0.5 | 0.7 | 0.3 | 0.4 | 0.7 |
| | | Oligomer (%) | ND | ND | ND | ND | ND |
| | 3C | Main peak (%) | 99.6 | 99.4 | 99.7 | 99.6 | 99.6 |
| | | Polymer (%) | 0.4 | 0.6 | 0.3 | 0.4 | 0.4 |
| | | Oligomer (%) | ND | ND | ND | ND | ND |
| | 5C | Main peak (%) | 99.3 | 99.4 | 99.7 | 99.5 | 99.6 |
| | | Polymer (%) | 0.5 | 0.6 | 0.3 | 0.4 | 0.4 |
| | | Oligomer (%) | 0.1 | 0 | ND | 0.1 | ND |

**Table 30. CE-SDS (NR) detection results at different temperatures/time points**

| Condition | Time point | CE-SDS(NR) | F3-2-1 | F3-2-2 | F3-2-3 | F3-2-4 | F3-3-1 |
|---|---|---|---|---|---|---|---|
| | T0 | Main peak (%) | 98.66 | 98.92 | 99.10 | 99.09 | 97.78 |
| | | Polymer (%) | ND | ND | ND | ND | ND |
| | | Oligomer (%) | 1.34 | 1.08 | 0.90 | 0.91 | 2.22 |
| | 4W | Main peak (%) | 97.55 | 97.58 | 97.69 | 97.71 | 97.53 |
| 2-8°C | | Polymer (%) | 0.17 | 0.18 | 0.18 | 0.18 | 0.21 |
| | | Oligomer (%) | 2.27 | 2.24 | 2.13 | 2.11 | 2.27 |
| | 3M | Main peak (%) | 97.16 | 97.19 | 96.99 | 96.93 | 97.26 |
| | | Polymer (%) | 0.07 | 0.05 | 0.06 | 0.07 | 0.07 |
| | | Oligomer (%) | 2.78 | 2.76 | 2.95 | 3.00 | 2.68 |
| | T0 | Main peak (%) | 98.66 | 98.92 | 99.10 | 99.09 | 97.78 |
| | | Polymer (%) | ND | ND | ND | ND | ND |
| | | Oligomer (%) | 1.34 | 1.08 | 0.90 | 0.91 | 2.22 |
| | 4W | Main peak (%) | 96.44 | 96.2 | 96.41 | 96.58 | 97.01 |
| 25°C | | Polymer (%) | 0.3 | 0.26 | 0.29 | 0.31 | 0.23 |
| | | Oligomer (%) | 3.26 | 3.54 | 3.3 | 3.12 | 2.77 |
| | 3M | Main peak (%) | 94.46 | 94.48 | 94.09 | 93.98 | 95.02 |
| | | Polymer (%) | 0.13 | 0.12 | 0.19 | 0.16 | 0.13 |
| | | Oligomer (%) | 5.41 | 5.40 | 5.72 | 5.86 | 4.85 |
| 40°C | T0 | Main peak (%) | 98.66 | 98.92 | 99.10 | 99.09 | 97.78 |
| | | Polymer (%) | ND | ND | ND | ND | ND |
| | | Oligomer (%) | 1.34 | 1.08 | 0.90 | 0.91 | 2.22 |
| | 1W | Main peak (%) | 97.27 | 97.18 | 97.04 | 97.07 | 97.4 |
| | | Polymer (%) | 0.24 | 0.2 | 0.34 | 0.29 | 0.15 |
| | | Oligomer (%) | 2.5 | 2.62 | 2.62 | 2.64 | 2.45 |
| | 2W | Main peak (%) | 94.44 | 94.19 | 94.56 | 94.48 | 95.47 |
| | | Polymer (%) | 0.48 | 0.44 | 0.60 | 0.66 | 0.34 |
| | | Oligomer (%) | 5.08 | 5.38 | 4.85 | 4.85 | 4.19 |
| | 4W | Main peak (%) | 92.14 | 92.27 | 92.38 | 92.29 | 93.5 |
| | | Polymer (%) | 0.54 | 0.58 | 0.79 | 0.69 | 0.31 |
| | | Oligomer (%) | 7.32 | 7.15 | 6.84 | 7.02 | 6.2 |
| | T0 | Main peak (%) | 98.66 | 98.92 | 99.10 | 99.09 | 97.78 |
| | | Polymer (%) | ND | ND | ND | ND | ND |
| | | Oligomer (%) | 1.34 | 1.08 | 0.90 | 0.91 | 2.22 |
| | 4D | Main peak (%) | 98.26 | 98.22 | 98.19 | 97.88 | 98.33 |
| Shaking test | | Polymer (%) | 0.14 | 0.17 | 0.21 | 0.15 | 0.15 |
| | | Oligomer (%) | 1.6 | 1.61 | 1.6 | 1.97 | 1.53 |
| | 7D | Main peak (%) | 98.06 | 98.12 | 98.14 | 98.11 | 98.16 |
| | | Polymer (%) | 0.18 | 0.13 | 0.18 | 0.19 | 0.18 |
| | | Oligomer (%) | 1.77 | 1.76 | 1.67 | 1.7 | 1.66 |
| | T0 | Main peak (%) | 98.66 | 98.92 | 99.10 | 99.09 | 97.78 |
| | | Polymer (%) | ND | ND | ND | ND | ND |
| | | Oligomer (%) | 1.34 | 1.08 | 0.90 | 0.91 | 2.22 |
| | 3C | Main peak (%) | 98.31 | 97.78 | 97.8 | 97.98 | 98.3 |
| Freezing-thawin g test | | Polymer (%) | 0.15 | 0.14 | 0.14 | 0.16 | 0.17 |
| | | Oligomer (%) | 1.53 | 2.08 | 2.05 | 1.86 | 1.54 |
| | 5C | Main peak (%) | 98.05 | 98.21 | 98.28 | 98.08 | 98.17 |
| | | Polymer (%) | 0.21 | 0.14 | 0.17 | 0.16 | 0.16 |
| | | Oligomer (%) | 1.73 | 1.65 | 1.54 | 1.76 | 1.68 |

**Table 31. CE-SDS (R) detection results at different temperatures/time points**

| Condition | Time point | CE-SDS(R) | F3-2-1 | F3-2-2 | F3-2-3 | F3-2-4 | F3-3-1 |
|---|---|---|---|---|---|---|---|
| 2-8°C | T0 | Main peak (%) | 99.47 | 99.39 | 98.98 | 96.86 | 99.15 |
| | | Other peaks (%) | 0.53 | 0.61 | 1.02 | 3.15 | 0.85 |
| | 4W | Main peak (%) | 99.44 | 99.63 | 99.61 | 99.46 | 99.57 |
| | | Other peaks (%) | 0.56 | 0.38 | 0.38 | 0.54 | 0.43 |
| | 3M | Main peak (%) | 98.90 | 98.87 | 98.45 | 98.61 | 98.90 |
| | | Other peaks (%) | 1.10 | 1.13 | 1.55 | 1.39 | 1.10 |
| 25°C | T0 | Main peak (%) | 99.47 | 99.39 | 98.98 | 96.86 | 99.15 |
| | | Other peaks (%) | 0.53 | 0.61 | 1.02 | 3.15 | 0.85 |
| | 4W | Main peak (%) | 99.16 | 99.29 | 98.90 | 99.22 | 99.55 |
| | | Other peaks (%) | 0.84 | 0.71 | 1.10 | 0.78 | 0.45 |
| | 3M | Main peak (%) | 97.24 | 97.30 | 96.53 | 96.50 | 97.56 |
| | | Other peaks (%) | 2.76 | 2.70 | 3.47 | 3.50 | 2.44 |
| 40°C | T0 | Main peak (%) | 99.47 | 99.39 | 98.98 | 96.86 | 99.15 |
| | | Other peaks (%) | 0.53 | 0.61 | 1.02 | 3.15 | 0.85 |
| | 1W | Main peak (%) | 97.90 | 98.33 | 97.14 | 97.14 | 98.41 |
| | | Other peaks (%) | 2.10 | 1.67 | 2.86 | 2.86 | 1.59 |
| | 2W | Main peak (%) | 96.94 | 96.94 | 96.76 | 96.8 | 97.85 |
| | | Other peaks (%) | 3.06 | 3.06 | 3.23 | 3.2 | 2.15 |
| | 4W | Main peak (%) | 95.74 | 95.64 | 95.13 | 94.95 | 97.33 |
| | | Other peaks (%) | 4.25 | 4.35 | 4.88 | 5.05 | 2.68 |
| Shaking test | T0 | Main peak (%) | 99.47 | 99.39 | 98.98 | 96.86 | 99.15 |
| | | Other peaks | 0.53 | 0.61 | 1.02 | 3.15 | 0.85 |
| | 4D | Main peak (%) | 99.17 | 99.18 | 99.00 | 99.02 | 99.26 |
| | | Other peaks (%) | 0.83 | 0.82 | 1.00 | 0.98 | 0.74 |
| | 7D | Main peak (%) | 99.13 | 99.16 | 98.96 | 98.91 | 99.23 |
| | | Other peaks (%) | 0.87 | 0.84 | 1.04 | 1.09 | 0.77 |
| Freezing-thawing test | T0 | Main peak (%) | 99.47 | 99.39 | 98.98 | 96.86 | 99.15 |
| | | Other peaks (%) | 0.53 | 0.61 | 1.02 | 3.15 | 0.85 |
| | 3C | Main peak (%) | 99.27 | 99.28 | 99.08 | 99.15 | 99.25 |
| | | Other peaks (%) | 0.74 | 0.72 | 0.92 | 0.85 | 0.76 |
| | 5C | Main peak (%) | 99.28 | 99.32 | 99.07 | 99.12 | 99.30 |
| | | Other peaks (%) | 0.73 | 0.68 | 0.93 | 0.89 | 0.69 |

### 3. Investigation results

This example investigated the protective effect of the surfactant, polysorbate 80 (PS80), on proteins in a pH 5.5 acetic acid system. According to the results, the proteins had good tolerance to the high temperature, repeated freezing and thawing, and shaking under the conditions of 20 mM acetic acid-sodium acetate, 140 mM arginine hydrochloride, 0.04% or 0.06% PS80 (w/v), and the concentration of 2 mg/mL and 5 mg/mL; the proteins also had good tolerance to high temperature, repeated freezing and thawing, and shaking under the conditions of 20 mM acetic acid-sodium acetate, 8% sucrose (w/v), 0.04% PS80 (w/v), and the protein concentration of 2 mg/mL. There was no significant difference among the formulas in appearance, pH, concentration, protein particle size (DLS), SEC-HPLC, and CE (NR); in formula F3-3-1, 8% sucrose was used as an excipient, the CE-SDS (R) results of this formula were better in protein purity at 40 °C for 4 W, and the proportion of other peaks was 2.68%, which was lower than those of other formulas (4.25%-5.05%), as compared with other formulas using arginine hydrochloride. In conclusion, PS80 was capable of effectively protecting proteins from shear forces in the tolerance environment and during production, preventing protein aggregation.

### 4. Druggability study

The stability of the 5 mg/mL IL-2 mutant fusion protein in the system of 20 mM acetic acid-sodium acetate, pH 5.2, 8% sucrose (w/v), and 0.02% PS80 (w/v) was investigated, and the results showed that the 5 mg/mL IL2 mutant fusion protein showed good druggability in the formula of druggability study, as detailed in Table 32.

**Table 32. Druggability study results**

| Condition | Appearance | Concentratio n | SEC-HPLC | | | CE-SDS (NR) | | |
|---|---|---|---|---|---|---|---|---|
| | | | Main peak (%) | Polymer (%) | Oligome r (%) | Main peak (%) | Polymer (%) | Oligome r (%) |
| T0 | Colorless and clear liquid | 5.099 | 100 | ND | ND | 98.78 | ND | 1.22 |
| 40°C,1W | Colorless and clear liquid | 5.119 | 99.41 | 0.32 | 0.27 | 97.57 | ND | 2.44 |
| 40°C,2W | Colorless and clear liquid | 5.192 | 99.15 | 0.05 | 0.71 | 95.14 | ND | 4.86 |
| 40°C,4W | Colorless and clear liquid | 5.146 | 99.4 | ND | 0.6 | 92.28 | ND | 7.72 |
| 2~8°C,4W | Colorless and clear liquid | 5.069 | 100 | ND | ND | 98.46 | ND | 1.54 |
| pH3.5, 1H | NA | 5.454 | 100 | ND | ND | 98.77 | ND | 1.22 |
| pH3.5, 3H | NA | 5.629 | 99.9 | 0.1 | ND | 98.75 | ND | 1.24 |
| 4500Lux, 25 °C , 5D | Colorless and clear liquid | 5.128 | 99.83 | 0.17 | ND | 98.25 | ND | 1.74 |
| Dark control, 25 °C, 5 D | Colorless and clear liquid | 5.160 | 99.86 | 0.14 | ND | 98.28 | ND | 1.72 |
| 4500Lux, 25°C, 10D | Colorless and clear liquid | 5.145 | 99.85 | 0.15 | ND | 98.38 | ND | 1.62 |
| Dark control, 25 °C, 10 D | Colorless and clear liquid | 5.114 | 99.97 | 0.03 | ND | 98.52 | ND | 1.48 |

### 5. Conclusion

Considering that formula F3-3-1 showed better results in CE-SDS (R) than other formulas when screening surfactants, and simultaneously considering that both 2 mg/mL and 5 mg/mL were low-concentration proteins and that the 5 mg/mL IL2 mutant fusion protein showed good stability in the druggability study of similar formula to F3-3-1, 5 mg/mL IL2 mutant fusion protein, 20 mM acetic acid-acetate, pH 5.5, 8% sucrose (w/v), and 0.04% PS80 (w/v) were selected as the target formula for the further formula stability confirmation study.

### Example 5. Formula Stability Confirmation

### 1. Formula stability confirmation protocol

The target formula (5 mg/mL IL2 mutant fusion protein, 20 mM acetic acid-sodium acetate, pH 5.5, 8% sucrose (w/v), and 0.04% PS80 (w/v)) was selected for the formula stability confirmation study. 1 mL of a liquid formulation was aliquoted into 2-mL vials; a rubber stopper and an aluminum-plastic cap were used to form a closed packaging system; and the vials were placed upright and inverted to investigate the stability of the formulation. The investigation protocol is shown in Table 33.

**Table 33. Formula stability confirmation protocol**

| Conditions | | T0 | 2W | 4W | 3M |
|---|---|---|---|---|---|
| 40 °C | Upright (U) | X | X | X | -- |
| | Inverted (I) | | | | |
| 25 °C | Upright (U) | | -- | X | X |
| | Inverted (I) | | | | |
| 2 ~ 8 °C | Upright (U) | | -- | -- | X |

| | | | | | |
|---|---|---|---|---|---|
| Note: X = appearance, pH value, protein concentration, icIEF, SEC-HPLC, CE-SDS (NR), CE-SDS (R), insoluble particle (MFI), and binding activity assay. | | | | | |

### 2. Formula stability confirmation results

The major results of the formula stability confirmation are summarized in the table below.

**Table 34-1. Formula stability confirmation results**

| Condition | | Detection time point | Appearance | Concentratio n (mg/mL) | pH | Insoluble particle (MFI, particles/mL) | |
|---|---|---|---|---|---|---|---|
| | | | | | | ≥ 10µm | ≥ 25µm |
| Quality criteria | | | Colorless to light yellow, transparent to slightly opalescent, substantially no visible particles | 5.0±0.5 | 5.5 ± 0.5 | ≤ 6000/vial | ≤ 600/vial |
| 40 °C | U | T0 | Colorless, clear, no visible particles | 4.79 | 5.5 | 10 | 0 |
| | | 2W | Colorless, clear, no visible particles | 4.81 | 5.5 | 23 | 10 |
| | | 4W | Colorless, clear, no visible particles | 4.83 | 5.5 | 7 | 3 |
| | I | T0 | Colorless, clear, no visible particles | 4.79 | 5.5 | 10 | 0 |
| | | 2W | Colorless, clear, no visible particles | 4.80 | 5.5 | 25 | 3 |
| | | 4W | Colorless, clear, no visible particles | 4.84 | 5.5 | 3 | 0 |
| 25 °C | U | T0 | Colorless, clear, no visible particles | 4.79 | 5.5 | 10 | 0 |
| | | 4W | Colorless, clear, no visible particles | 4.81 | 5.5 | 14 | 0 |
| | | 3M | Colorless, clear, no visible particles | 4.83 | 5.5 | 19 | 5 |
| | I | T0 | Colorless, clear, no visible particles | 4.79 | 5.5 | 10 | 0 |
| | | 4W | Colorless, clear, no visible particles | 4.81 | 5.5 | 3 | 0 |
| | | 3M | Colorless, clear, no visible particles | 4.83 | 5.5 | 35 | 3 |
| 2~8 °C | U | T0 | Colorless, clear, no visible particles | 4.79 | 5.5 | 10 | 0 |
| | | 3M | Colorless, clear, no visible particles | 4.82 | 5.5 | 12 | 3 |

**Table 34-2. Formula stability confirmation results**

| Condition | | Detection time point | icIEF (%) | | | SEC-HPLC (%) | CE-SDS (NR) (%) | CE-SDS (R) (%) | Activity (%) |
|---|---|---|---|---|---|---|---|---|---|
| | | | Acid peak | Main peak | Base peak | Monomer | Main peak | Main peak | ELISA |
| Quality criteria | | | Report result | ≥50.0 | Report result | ≥92% | ≥90% | ≥92% | 70 - 130 |
| 40 °C | U | T0 | 21.5 | 78.3 | 0.3 | 99.5 | 98.6 | 98.74 | 108 |
| | | 2W | 23.9 | 75.3 | 0.7 | 98.6 | 96.3 | 98.41 | 92 |
| | | 4W | 30.3 | 69.1 | 0.6 | 98.6 | 94.3 | 95.22 | 103 |
| | I | T0 | 21.5 | 78.3 | 0.3 | 99.5 | 98.6 | 98.74 | 108 |
| | | 2W | 24.2 | 75.1 | 0.7 | 98.9 | 96.1 | 98.39 | 98 |
| | | 4W | 30.3 | 69.1 | 0.6 | 98.6 | 94.0 | 95.54 | 95 |
| 25 °C | U | T0 | 21.5 | 78.3 | 0.3 | 99.5 | 98.6 | 98.74 | 108 |
| | | 4W | 20.2 | 79.2 | 0.5 | 99.3 | 97.7 | 99.16 | 116 |
| | | 3M | 23.0 | 76.5 | 0.5 | 99.4 | 96.4 | 97.31 | 102 |
| | I | T0 | 21.5 | 78.3 | 0.3 | 99.5 | 98.6 | 98.74 | 108 |
| | | 4W | 20.2 | 79.2 | 0.6 | 99.4 | 97.9 | 99.13 | 99 |
| | | 3M | 23.4 | 76.1 | 0.6 | 99.3 | 96.4 | 97.59 | 105 |
| 2~8 °C | U | T0 | 21.5 | 78.3 | 0.3 | 99.5 | 98.6 | 98.74 | 108 |
| | | 3M | 20.4 | 79.3 | 0.3 | 99.8 | 98.3 | 99.03 | 95 |

### 3. Conclusion of formula stability confirmation study

The samples were placed at the low temperature of 2-8 °C for 3 months and subjected to accelerated stability investigation at 25 °C for 3 months. The upright and inverted samples had no significant change in conventional detection indexes, insoluble particle, purity, charge heteroplasmon, activity, and the like, and there was no significant difference between the upright and inverted samples. After 4 weeks of accelerated stability investigation at 40 °C, the main peak of the charge variant was decreased by 9.2%, mainly tending to the acid peak value, and the purity and charge variant were significantly changed.

In conclusion, in the investigation process of low-temperature (2-8 °C) real-time stability and 25 °C accelerated test, the samples had no significant change in conventional detection indexes, insoluble particle, purity, charge heteroplasmon, activity, and the like, and all indexes were within the quality criteria, indicating that the IL2 mutant fusion protein has better stability in this formula. In the high-temperature 40 °C accelerated stability investigation, the purity and charge heteroplasmon had a downward trend, indicating that the formulation of the present invention is sensitive to high temperature.

### Example 6. Single-Drug Pharmacodynamic Study of IL2 Mutant Fusion Protein Injections in DTH Model Mice

In this example, the single-drug efficacy of the IL2 mutant fusion protein injections was evaluated on KLH-induced delayed-type hypersensitivity (DTH) model mice. The IL2 mutant fusion protein was IL2 mutant 1-2-linker-Fc (V91R/Y31V/A73L/H79Q), hereinafter referred to as IL2-1-2, the sequence of which is set forth in SEQ ID NO: 15. Fc-linker-IL2 mutant 6 (V91K), i.e., AMG592, was used as a positive control, the sequence of which is set forth in SEQ ID NO: 20. The injection formulas of IL2-1-2 and AMG592 were all 5 mg/mL IL2-1-2 or AMG592, 20 mM acetic acid-sodium acetate, pH 5.5, 8% sucrose (w/v), and 0.04% PS80 (w/v).

### 6.1 Animal grouping

BALB/c mice, 6-8 weeks old, were used, and each mouse was weighed. The mice were randomly divided and distributed into 8 experimental groups G0-G7, 10 mice per group, according to the body weight of mice. The administration was started on the day of grouping, which was defined as Day 0. The mice in each dosage group were subcutaneously injected with IL2-1-2 and AMG592 once every three days, and intraperitoneally injected with CsA once every day until the end of the experiment.

### 6.2 Animal model construction protocol

### 6.2.1 Reagent preparation

(1) 3 mg/mL KLH: a lyophilized powder was weighed out and prepared into a 3 mg/mL KLH solution with PBS.
(2) 1 mg/mL KLH emulsion: antigens were emulsified with KLH (3 mg/mL), IFA, and CFA in a volume ratio of 1:1:1 by using the communicating tube syringe method, so that the antigen could be fully emulsified to form a viscous emulsion.
(3) 1 mg/mL KLH solution: 3 mg/mL KLH was 3-fold diluted to 1 mg/mL with PBS. 6.2.2 DTH molding method

BALB/c mice were injected with 100 µL of KLH emulsion (emulsified by KLH, IFA, and CFA in a volume ratio of 1:1:1) with a concentration of 1 mg/mL at two points on the right side of the back on day 0 for sensitization. Mice in the normal control group were injected with an equal volume of emulsion without KLH. On day 5, 10 µL of KLH solution with a concentration of 1 mg/mL was intradermally injected into the right ear of each mouse to stimulate the mice, thereby producing symptoms of delayed-type hypersensitivity in the local skin tissues of the model mice. The thickness of the right ear of the mice was measured using a micrometer before and 24 h, 48 h, 72 h, and 96 h after the local injection of KLH in the right ear, so as to evaluate the degree of local delayed-type hypersensitivity in mice of different treatment groups.

### 6.3 Grouping and administration regimens are shown in Table 35

**Table 35. Administration regimens of IL2 mutant fusion protein injections in DTH model mice**

| **Group** | **Number of animals** | **Test compound** | **Molding** | **Dose (mg/kg)^{a}** | **Route of administration** | **Frequency of administratio n** | **Number of administratio n** |
|---|---|---|---|---|---|---|---|
| G0 | 10 | N/A | N/A | N/A | N/A | N/A | N/A |
| G1 | 10 | Vehicle | YES | N/A | s.c. | q3d | 3 |
| G2 | 10 | CsA | YES | 10 | i.p. | qd | 3 |
| G3 | 10 | AMG592 | YES | 1 | s.c. | q3d | 3 |
| G4 | 10 | IL2-1-2 | YES | 1 | s.c. | q3d | 3 |
| G5 | 10 | IL2-1-2 | YES | 0.3 | s.c. | q3d | 3 |
| G6 | 10 | IL2-1-2 | YES | 0.1 | s.c. | q3d | 3 |
| G7 | 10 | IL2-1-2 | YES | 0.03 | s.c. | q3d | 3 |

### 6.4 Detection indexes

6.4.1 Weight detection: the animals were weighed before grouping, and the mice were weighed once every three days after grouping. The data were recorded with one decimal place reserved by taking g as a unit.

### 6.4.2 Measurement of mouse ear thickness:

(1) Before stimulation, the thickness of the right ear of each mouse was measured using a digital outside micrometer to be used as a background value
(2) After stimulation, mouse ear thickness was measured at 24 h, 48 h, 72 h, and 96 h.

6.4.3 General clinical observation: the mice were observed at least 2 times every week during the acclimatization and during the experiment. The observations include, but are not limited to, animals' mental states, diet condition, and the like. If unexpected conditions occurred, the record must be carried out on an experimental record book (paper).

### 6.5 Termination of experiment

6.5.1 The animals should be observed for the health status at the interval of administration, and if any one or more of the following conditions occurred, the administration should be suspended until the animals returned to normal:
(1) The administration was stopped when the body weight of the animals was less than 81% of the body weight at the beginning of drug treatment, and the administration was performed again until the body weight returned to 90% of the body weight at the beginning of drug treatment; and
(2) after administration, the animals showed slow or abnormal movements, or had an acute stress phenomenon.

6.5.2 Humane endpoint for experimental animals: during the experiment, when the state of an animal was abnormal, the health status of the animal was evaluated to determine whether to provide treatment, whether to continue the experiment, or whether to perform euthanasia.

6.5.3 Euthanasia: 96 h after stimulation of mice, animals were euthanized by asphyxiation with excess CO₂ at the end of the experiment or at the humane endpoint.

### 6.6 Statistical analysis method

The raw data of measurements and observations must be recorded. Analysis and processing were performed on the basis of the raw data, and the analysis results were expressed as mean ± standard error (Mean ± SEM). Meanwhile, statistical analysis was performed on the data, Two-way ANOVA was adopted to analyze the thickness change of the ears of the mice, One-way ANOVA was adopted to analyze the body weight of the mice on day 10, and P < 0.05 was considered to have statistical significance.

### 6.7 Results and discussion

### 6.7.1 Effects of IL2-1-2 injections on mouse body weight in DTH model

The body weight of the experimental animal was used as a reference index for indirectly determining the toxicity of the drug. The body weights and body weight change rates of the mice after administration of CsA, AMG592, and different doses of IL2-1-2 are shown in FIGs. 1 and 2 and Table 36. The DTH model mice were given therapeutic drugs by subcutaneous injection once every three days. During the observation period, the body weights of the mice fluctuated, except that the body weight of the mice in the hormone-administered CsA group was decreased, but the body weight of the mice was not decreased by more than 10%, and the body weights of the mice in other administration groups showed an increasing trend. All mice were not abnormal in status, and no other morbidity or mortality. Therefore, it could be seen that the treatments were all tolerated at doses of 10 mg/kg CsA, 1 mg/kg AMG592, and 0.03 mg/kg, 0.1 mg/kg, 0.3 mg/kg, and 1 mg/kg IL2 mutant 1-2.

**Table 36. Effects of mutant 1-2 injections on body weight of DTH model mice**

| **Group** | **Number of animals Start/end point** | **Mean body weight (g)^{a} (Day 0)** | **Mean body weight (g)^{a} (Day 10)** | **Body weight change (%) (Day 10)** |
|---|---|---|---|---|
| Normal control | 10/10 | 18.2±0.27 | 18.7±0.31 | 2.78 |
| Vehicle control | 10/10 | 18.4±0.21 | 18.5±0.27 | 0.58 |
| CSA 10mg/kg | 10/10 | 18.4±0.20 | 18.0±0.32 | -1.92 |
| AMG592, 1mg/kg | 10/10 | 18.3±0.22 | 19.2±0.14 | 4.54 |
| IL2-1-2, 1mg/kg | 10/10 | 18.3±0.21 | 18.8±0.15 | 2.87 |
| IL2-1-2, 0.3mg/kg | 10/10 | 18.3±0.19 | 19.7±0.24** | 7.63 |
| IL2-1-2, 0.1mg/kg | 10/10 | 18.3±0.24 | 18.9±0.34 | 3.44 |
| IL2-1-2, 0.03mg/kg | 10/10 | 18.3±0.22 | 18.9±0.20 | 2.94 |

| | | | | |
|---|---|---|---|---|
| a: mean ± standard error (SEM), **P < 0.005. | | | | |

### 6.7.2 Effects of IL2-1-2 injections on thickness change of ears of DTH model mice

The change in ear thickness of the DTH model mice in each treatment group is shown in FIGs. 3-4. The disease onset of the DTH model reached a peak 48 h after KLH stimulation, the change value of the ear thickness of the mice in the model group at the peak of the disease onset was 15.7 mm × 10⁻², which was increased by 74.1% compared with the initial ear thickness of the mice. The model was successfully constructed. In the treatment groups of 1 mg/kg, 0.3 mg/kg, 0.1 mg/kg, and 0.03 mg/kg of IL2-1-2 injections, the ear thicknesses of the mice were increased by 2.45 mm × 10⁻², 4.01 mm × 10⁻², 9.09 mm × 10⁻², and 12.26 mm × 10⁻², which were changed by 10.2%, 22.0%, 41.3%, and 53.6% compared with the ear thickness of the mice before KLH stimulation. The positive drugs, 10 mg/kg CsA and 1 mg/kg AMG592, also had significant therapeutic effects on the model, and showed certain therapeutic effects (P < 0.0001) compared with the vehicle control group, but the therapeutic effects of these two drugs were not as good as those of 0.3 mg/kg and 1 mg/kg IL2-1-2. In conclusion, IL2-1-2 has a significant therapeutic effect on KLH-induced DTH model mice.

### Example 7. Pharmacodynamic Study of IL2 Mutant Fusion Protein Injections in BALB/c Mice

In this example, the pharmacodynamic properties of the IL2 mutant fusion protein injections were evaluated on BALB/c mice. The IL2 mutant fusion protein was IL2 mutant 1-2-linker-Fc (V91R/Y31V/A73L/H79Q), hereinafter referred to as IL2-1-2, the sequence of which is set forth in SEQ ID NO: 15. Fc-linker-IL2 mutant 6 (V91K), i.e., AMG592, was used as a positive control, the sequence of which is set forth in SEQ ID NO: 20. The injection formulas of IL2-1-2 and AMG592 were all 5 mg/mL IL2-1-2 or AMG592, 20 mM acetic acid-sodium acetate, pH 5.5, 8% sucrose (w/v), and 0.04% PS80 (w/v).

### 7.1 Animal grouping

After 6-8 week-old BALB/c mice were acclimatized, each mouse was weighed. According to the body weight of mice, the mice were randomly divided and distributed into 5 experimental groups G1-G5 using EXCEL based on random numbers, 5 mice per group. The administration was started on the day of grouping, which was defined as Day 1.

### 7.2 Grouping and administration regimens are shown in Table 37.

**Table 37. Administration regimens of IL2 mutant fusion protein injections in BALB/c mice**

| **Group** | **Number of animals** | **Treatmen t** | **Dose (mg/kg)** | **Mode of administratio n** | **Number of administratio n** | **Blood sampling time point** |
|---|---|---|---|---|---|---|
| **G1** | 5 | Vehicle | N/A | NA | NA | Day 4 |
| **G2** | 5 | IL2-1-2 | 0.1 | s.c | Single (Day 1) | Day 4 |
| **G3** | 5 | IL2-1-2 | 0.3 | s.c | Single (Day 1) | Day 4 |
| **G4** | 5 | IL2-1-2 | 1 | s.c | Single (Day 1) | Day 4 |
| **G5** | 5 | AMG592 | 1 | s.c | Single (Day 1) | Day 4 |

### 7.3 Detection indexes

7.3.1 Weight detection: the animals were weighed before grouping, and the mice were weighed after grouping and before blood sampling., with g as a unit.

### 7.3.2 Immune cell typing in mouse peripheral blood by flow cytometry

On day 4 after administration, peripheral blood was collected by cardiac puncture after the mice were euthanized. Immune cell surface markers (CD3+, CD4+, and CD25+) and nuclear factors (Foxp3+ and Ki-67+) were labeled with fluorescent label antibodies. Immune cell counting and clustering analysis were performed by the FACS method as follows:
(1) After the mice were euthanized at the end point, the heart was punctured, and peripheral blood was collected into an EDTA-2K anticoagulant tube. The anticoagulant tube was gently inverted to fully mix the blood with the anticoagulant to prevent coagulation. (2) 300 µL of uncoagulated whole blood was transferred into a flow tube. The antibody mixture of a cell surface marker (CD3+, CD4+, or CD25+) was added to the flow tube, and the resulting mixture was incubated at room temperature for 20 min in the dark. (3) 1 mL of lysis buffer was added to the flow tube to lyse the red blood cells, and the resulting mixture was incubated at room temperature for 5 min in the dark. The flow tube was centrifuged at 20 °C and 400 g/rcf for 6 min. The supernatant was discarded. (4)

Step (3) was repeated, and the red blood cells were lysed again. (5) The cells were resuspended in 4 mL of wash buffer (PBS). The cell suspension was filtered through a filter membrane and transferred to a new flow tube. (6) 500 µL of fixative solution was added dropwise to the flow tube while shaking, and the resulting mixture was incubated at 4 °C overnight in the dark. (7)2 mL of permeabilization solution was added to the flow tube and shaken, and the flow tube was centrifuged at 4 °C and 500 g/rcf for 5 min. The supernatant was discarded. (8) Step (7) was repeated, and the cells were treated again with 3 mL of the permeabilization solution. (9) The antibody mixture of a nuclear factor (Foxp3+ or Ki-67+) was added to the flow tube, and the resulting mixture was incubated at 4 °C for 40 min in the dark. The flow tube was shaken every 20 min. (10) 4 mL of wash buffer was added to the flow tube and shaken, and the flow tube was centrifuged at 4 °C and 500 g/rcf for 5 min. The supernatant was discarded, and the cells were resuspended in 200 µL of PBS. (11) Counting beads were vortexed and equilibrated at room temperature for at least 30 s. 50 µL of counting beads were added to the flow tubes, and FACS assay was performed on a flow cytometer. Each flow tube needed to be fully shaken before loading. (12)The types of immune cells were analyzed by the FlowJo software according to the FACS results, and the cells were counted as indicated by counting beads.

7.3.3 General clinical observation: the mice were observed at least 2 times every week during the acclimatization and during the experiment. The observations include, but are not limited to, animals' mental states, diet condition, and the like. If unexpected conditions occurred, the record must be carried out on an experimental record book (paper).

### 7.4 Termination of experiment

7.4.1 The animals should be observed for the health status at the interval of administration, and if any one or more of the following conditions occurred, the administration should be suspended until the animals returned to normal:
(1) The administration was stopped when the body weight of the animals was less than 81% of the body weight at the beginning of drug treatment, and the administration was performed again until the body weight returned to 90% of the body weight at the beginning of drug treatment; and
(2) after administration, the animals showed slow or abnormal movements, or had an acute stress phenomenon.

7.4.2 Humane endpoint for experimental animals: during the experiment, when the state of an animal was abnormal, the health status of the animal was evaluated to determine whether to provide treatment, whether to continue the experiment, or whether to perform euthanasia.

7.4.3 Euthanasia: animals were euthanized by asphyxiation with excess CO₂ at the experimental endpoint of day 26 after sensitization.

### 7.5 Statistical analysis method

The raw data of measurements and observations must be recorded. Analysis and processing were performed on the basis of the raw data, and the analysis results were expressed as mean ± standard error (Mean ± SEM).

### 7.6 Results and discussion

7.6.1 Effects of IL2-1-2 injections on immune cells in peripheral blood of BALB/c mice After the BALB/c mice were treated with different doses of IL2-1-2, the fold change in immune cell count in the peripheral blood compared to the mice in the vehicle control group is shown in Table 38. It could be seen that subcutaneous injection of IL2-1-2 had a significant promotion effect on the proliferation of mouse Treg cells. After 3 days of administration of IL2-1-2 or AMG592, Treg cells were significantly stimulated compared to the vehicle control group. After subcutaneous administration of 0.1 mg/kg, 0.3 mg/kg, and 1 mg/kg IL2-1-2, the fold change in Treg cell count in the mouse peripheral blood was 13.73, 35.27, and 12.05, respectively. The increase in Treg cell count of the mice in the 1 mg/kg AMG592 administration group was higher than that of the mice in the 1 mg/kg IL2-1-2 administration group, but was lower than that of the mice in the 0.3 mg/kg administration group. The results indicated that IL2-1-2 had a relatively good promotion effect on Treg cells only at a relatively low dose. The fold change in the Treg/CD4+ ratio is shown in Table 39. In the experiment, it was observed that CD4+ Foxp3 cells or CD3+ CD4- cells were not activated or slightly activated.

**Table 38. Fold change in immune cell count in peripheral blood**

| **Cell subsets** | **Day** | **Vehicle** | **IL2-1-2 0.1 mg/kg s.c.** | **IL2-1-2 0.3 mg/kg s.c.** | **IL2-1-2 1 mg/kg s.c.** | **AMG 592 1 mg/kg s.c.** |
|---|---|---|---|---|---|---|
| **Treg** | D4 | 1.00 ± 0.11 | 13.73 ± 1.35 | 35.27 ± 5.10 | 12.05 ± 1.98 | 28.12 ± 3.86 |
| **CD4**⁺ **Foxp3**⁻ | D4 | 1.00 ± 0.015 | 1.36 ± 0.40 | 1.29 ± 0.24 | 0.53 ± 0.09 | 1.53 ± 0.32 |
| **CD3⁺CD4⁻** | D4 | 1.00 ± 0.13 | 1.57 ± 0.39 | 2.25 ± 0.38 | 0.73 ± 0.15 | 2.57 ± 0.44 |
| **Ki-67**⁺ **Treg** | D4 | 1.00 ± 0.11 | 19.51 ± 1.68 | 54.88 ± 7.07 | 18.76 ± 3.56 | 34.52 ± 4.73 |

**Table 39. Fold change in Treg/CD4+ ratio**

| **Cell subsets** | **Day** | **Vehicle** | **IL2-1-2 0.1 mg/kg s.c.** | **IL2-1-2 0.3 mg/kg s.c.** | **IL2-1-2 1 mg/kg s.c.** | **AMG 592 1 mg/kg s.c.** |
|---|---|---|---|---|---|---|
| **Treg** | D4 | 1.00 ± 0.12 | 6.21 ± 0.58 | 9.17 ± 0.15 | 8.69 ± 0.41 | 8.01 ± 0.27 |

### 7.7 Conclusion

The results indicated that the subcutaneous injection of the IL2-1-2 injection could significantly promote the proliferation of Treg cells in the peripheral blood of BALB/c mice, and the mice were significantly tolerant to the administration doses of the test molecules.

### Example 8. Pharmacodynamic Study of IL2 Mutant Fusion Protein Injections in SD Rats

In this example, the pharmacodynamic properties of the IL2 mutant fusion protein injections were evaluated on SD rats. The IL2 mutant fusion protein was IL2 mutant 1-2-linker-Fc (V91R1Y31V/A73L/H79Q), hereinafter referred to as IL2-1-2, the sequence of which is set forth in SEQ ID NO: 15. The injection formulas of IL2-1-2 were all 5 mg/mL IL2-1-2, 20 mM acetic acid-sodium acetate, pH 5.5, 8% sucrose (w/v), and 0.04% PS80 (w/v).

### 8.1 Animal grouping

SD rats, 6-8 weeks old, were acclimatized and then randomly distributed into 6 experimental groups of 3 males and 3 females per group. The administration was started on the day of grouping (defined as day 1), and IL2-1-2 injections were administered 4 times by subcutaneous or tail vein injection on day 1, day 8, day 15, and day 22.

### 8.2 Grouping and administration regimens are shown in Table 40.

**Table 40. Administration regimens of IL2 mutant fusion protein injections in SD rats**

| **Group** | **Number of animals (male) (n)** | **Number of animals (female) (n)** | **Sample** | **Dose (mg/kg)** | **Mode of administrati on** | **Blood sampling time points** |
|---|---|---|---|---|---|---|
| G1 | 3 | 3 | Vehicle | 0 | s.c. | Day 1, 4, 11, 25 |
| G2 | 3 | 3 | IL2-1-2 | 0.005 | s.c. | Day 1, 4, 11, 25 |
| G3 | 3 | 3 | IL2-1-2 | 0.05 | s.c. | Day 1, 4, 11, 25 |
| G4 | 3 | 3 | IL2-1-2 | 1 | s.c. | Day 1, 4, 11, 25 |
| G5 | 3 | 3 | IL2-1-2 | 0.002 | i.v. | Day 1, 4, 11, 25 |
| G6 | 3 | 3 | IL2-1-2 | 1 | i.v. | Day 1, 4, 11, 25 |

| | | | | | | |
|---|---|---|---|---|---|---|
| Note: s.c.: subcutaneous injection; i.v.: intravenous injection | | | | | | |

### 8.3 Observation indexes

### 8.3.1 Body weight monitoring

The animals were weighed after grouping and before each administration and recorded in g.

### 8.3.2 Immune cell phenotype in rat peripheral blood by flow cytometry

Peripheral blood was collected from rats on day 1 before administration and on day 4, day 11, and day 25 after administration. Immune cell surface markers (CD3+, CD4+, and CD25+) and nuclear factors (Foxp3+ and Ki-67+) were labeled with fluorescent label antibodies. The number of immune cells was calculated by the fluorescence-activated cell sorting (FACS) method below:
(1) Whole blood was collected from the jugular vein of rats into an anticoagulant tube containing EDTA-2K. The anticoagulant tube was gently inverted to fully mix the blood with the anticoagulant to prevent coagulation. (2) 300 µL of uncoagulated whole blood was transferred into a flow tube. The antibody mixture of a cell surface marker (CD3+, CD4+, or CD25+) was added to the flow tube, and the resulting mixture was incubated at room temperature for 20 min in the dark. (3) 1 mL of lysis buffer was added to the flow tube to lyse the red blood cells, and the resulting mixture was incubated at room temperature for 5 min in the dark. The flow tube was centrifuged at 20 °C and 400 g/rcf. The supernatant was discarded. (4) Step (3) was repeated, and the red blood cells were lysed again. (5) The cells were resuspended in 4 mL of wash buffer (PBS). The cell suspension was filtered through a filter membrane and transferred to a new flow tube. (6) 500 µL of fixative solution was added dropwise to the flow tube while shaking, and the resulting mixture was incubated at 4 °C overnight in the dark. (7) 2 mL of permeabilization solution was added to the flow tube and shaken, and the flow tube was centrifuged at 4 °C and 500 g/rcf for 5 min. The supernatant was discarded. (8) Step (7) was repeated, and the cells were treated again with 3 mL of the permeabilization solution. (9) The antibody mixture of a nuclear factor (Foxp3+ or Ki-67+) was added to the flow tube, and the resulting mixture was incubated at 4 °C for 40 min in the dark. The flow tube was shaken every 20 min. (10) 4 mL of wash buffer was added to the flow tube and shaken, and the flow tube was centrifuged at 4 °C and 500 g/rcf for 5 min. The supernatant was discarded, and the cells were resuspended in 200 µL of PBS. (11) Counting beads were vortexed and equilibrated at room temperature for at least 30 s. 50 µL of counting beads were added to the flow tubes, and FACS assay was performed on a flow cytometer. Each flow tube needed to be fully shaken before loading. (12) The types of immune cells were analyzed by the FlowJo software according to the FACS results, and the cells were counted as indicated by counting beads.

### 8.3.3 General clinical observation

The rats were clinically observed at least 2 times every week during the acclimatization and during the experiment. The observations include, but are not limited to, animals' health status, performance, daily activities, mental states, diet condition, and the like. If unexpected conditions occurred, the record must be carried out on an experimental record book (paper).

### 8.4 Termination of experiment

8.4.1 The animals should be observed for the health status during the experiment and at the interval of administration. If any one or more of the following conditions occurred, the administration should be suspended until the animals returned to normal:
(1) The administration should be stopped when the body weight of the animals was less than 81% of the body weight before drug treatment, and the administration could be performed again until the body weight returned to 90% of the body weight before drug treatment; and (2) after administration, the animals showed slow or abnormal movements, or had an acute stress phenomenon.

### 8.4.2 Humane endpoint

The health status of the animal was evaluated to determine whether treatment or experiment should be performed or not, or whether euthanasia should be performed or not when any one or more of the following conditions occurred:
(1) movements of the animal were abnormal or the animal was paralyzed;
(2) the body weight of the animal was less than 20% of the body weight before grouping; and
(3) the animal was hypothermic, in a moribund state, or the like.

### 8.4.3 Euthanasia

At the humane endpoint or at the end of the experiment, animals were euthanized with excess CO₂.

### 8.5. Statistical analysis

The analysis results were expressed as mean ± standard error (Mean ± SEM).

### 8.6 Results

8.6.1 Effects of IL2-1-2 on the number and ratio of immune cells in rat PBMCs The changes in the number of immune cells in the peripheral blood of SD rats after IL2-1-2 administration are shown in Table 41. IL2-1-2 could cause expansion of Treg cells after subcutaneous and tail vein injection into SD rats. Compared with the control, a low dose of IL2-1-2 (0.005 mg/kg subcutaneous injection or 0.002 mg/kg tail vein injection) showed a slight promotion effect on the expansion of Treg cells from day 4 to day 25 after administration. After subcutaneous injection of a medium dose of IL2-1-2 (0.05 mg/kg), the number of Treg cells in the peripheral blood of rats increased to 9.55 times the baseline value on day 4 and maintained at 3.16 times the baseline value on day 25. After subcutaneous injection of a high dose of IL2-1-2 (1 mg/kg), the number of Treg cells in the peripheral blood of rats reached a peak value (13.8 times the baseline value) on day 11 and decreased to 3.18 times the baseline value on day 25. After tail vein injection of a high dose of IL2-1-2 (1 mg/kg), the numbers of Treg cells in the peripheral blood of rats significantly increased on both day 4 and day 11, which were 21.25 times and 30.13 times the baseline value, respectively. However, similarly to the other treatment groups, the number of Treg cells also decreased to 3.26 times the baseline value on day 25. This phenomenon was probably caused by the production of anti-drug antibodies (ADAs) in rats after multiple administrations. The expansion pattern of Ki-67+ cells in the Treg cells was also consistent with the trend of increasing Treg cell numbers.

The changes in the ratio of Treg/CD4+ cells are shown in Table 42. A low dose (0.005 mg/kg subcutaneous injection or 0.002 mg/kg tail vein injection) and a medium dose (0.05 mg/kg subcutaneous injection) of IL2-1-2 had no or slight activation effect on the numbers of CD4+ Foxp3- cells and CD3+ CD4- cells in the peripheral blood of rats, but a high dose (1 mg/kg subcutaneous injection or 1 mg/kg tail vein injection) of IL2-1-2 was observed to slightly activate the expansion of these cells described above.

**Table 41. Changes in cell number (baseline value as standard)**

| **Cell subpopulation** | **Date** | **Vehicle** | **0.005 mg/kg s.c.** | **0.05 mg/kg s.c.** | **1 mg/kg s.c.** | **0.002 mg/kg i.v.** | **1 mg/kg i.v.** |
|---|---|---|---|---|---|---|---|
| **Treg** | D4 | 1.10 ± 0.15 | 1.67 ± 0.23 | 9.55 ± 0.75 | 3.66 ± 0.67 | 1.59 ± 0.22 | 21.25 ± 4.53 |
| | D11 | 1.66 ± 0.23 | 1.90 ± 0.22 | 4.98 ± 1.34 | 13.80 ± 4.5 | 2.67 ± 0.39 | 30.13 ± 2.69 |
| | D25 | 1.69 ± 0.40 | 2.31 ± 0.54 | 3.16 ± 0.80 | 3.18 ± 0.90 | 2.15 ± 0.56 | 3.26 ± 0.60 |
| **CD4**⁺ **Foxp3**⁻ | D4 | 0.98 ± 0.09 | 1.20 ± 0.20 | 1.12 ± 0.09 | 0.65 ± 0.12 | 1.37 ± 0.18 | 2.45 ± 0.70 |
| **CD3**⁺ | D11 | 1.21 ± 0.31 | 1.42 ± 0.21 | 1.34 ± 0.22 | 2.17 ± 0.85 | 2.11 ± 0.36 | 3.17 ± 0.56 |
| | D25 | 0.97 ± 0.20 | 1.20 ± 0.22 | 0.77 ± 0.12 | 1.46 ± 0.48 | 1.37 ± 0.35 | 1.07 ± 0.17 |
| | D4 | 0.96 ± 0.10 | 1.18 ± 0.18 | 1.47 ± 0.11 | 1.27 ± 0.25 | 1.30 ± 0.17 | 8.70 ± 4.18 |
| **CD4**⁻ | D11 | 1.45 ± 0.22 | 1.42 ± 0.19 | 1.68 ± 0.26 | 8.28 ± 4.33 | 2.17 ± 0.44 | 16.98 ± 6.03 |
| | D25 | 1.08 ± 0.26 | 1.26 ± 0.23 | 0.89 ± 0.18 | 3.04 ± 1.17 | 1.40 ± 0.41 | 2.26 ± 0.37 |
| **Ki-67**⁺ **Treg** | D4 | 1.35 ± 0.29 | 2.38 ± 0.26 | 25.62 ± 2.25 | 10.59 ± 2.42 | 2.29 ± 0.42 | 69.38 ± 19.93 |
| | D11 | 1.87 ± 0.17 | 2.36 ± 0.22 | 9.93 ± 3.91 | 33.61 ± 9.87 | 3.49 ± 0.63 | 88.80 ± 10.84 |
| | D25 | 1.87 ± 0.57 | 2.69 ± 0.63 | 5.69 ± 1.72 | 3.61 ± 0.98 | 2.81 ± 0.85 | 4.09 ± 1.01 |

| | | | | | | | |
|---|---|---|---|---|---|---|---|
| Note: the results are expressed as mean ± standard error | | | | | | | |

**Table 42. Changes in Treg/CD4+ cell ratio**

| **Cell subpopulation** | **Date** | **Vehicle** | **0.005 mg/kg s.c.** | **0.05 mg/kg s.c.** | **1 mg/kg s.c.** | **0.002 mg/kg i.v.** | **1 mg/kg i.v.** |
|---|---|---|---|---|---|---|---|
| **Treg/CD4**⁺ | D4 | 1.11 ± 0.08 | 1.41 ± 0.09 | 6.06 ± 0.29 | 4.96 ± 0.99 | 1.16 ± 0.04 | 6.61 ± 0.48 |
| | D11 | 1.15 ± 0.07 | 1.37 ± 0.11 | 3.53 ± 0.94 | 4.98 ± 0.50 | 1.28 ± 0.06 | 6.94 ± 0.68 |
| | D25 | 1.61 ± 0.14 | 1.79 ± 0.16 | 3.42 ± 0.53 | 2.13 ± 0.14 | 1.52 ± 0.10 | 2.86 ± 0.49 |

| | | | | | | | |
|---|---|---|---|---|---|---|---|
| Note: the results are expressed as mean ± standard error | | | | | | | |

### 8.7 conclusion

The above experimental results show that IL2-1-2 could cause the proliferation of Treg cells by subcutaneous injection and tail vein injection into SD rats, and the SD rats showed good tolerance to IL2-1-2 at various doses.

## Claims

1. A pharmaceutical composition, wherein the pharmaceutical composition comprises: a fusion protein comprising an IL2 mutant and an antibody Fc block, a buffer, an osmotic pressure regulator, and a surfactant;
the fusion protein sequentially comprises, from the N-terminus to the C-terminus:
(1) the IL2 mutant, a linker, and the antibody Fc block, or
(2) the antibody Fc block, a linker, and the IL2 mutant;
preferably, the IL2 mutant comprises at least Y31V, A73L, and H79Q mutations compared to wild-type IL2;
more preferably, the IL2 mutant further comprises one or more of a V91R mutation, an H16E mutation, and a D20A mutation, for example, an H16E mutation and a V91R mutation;
the amino acid sequence of the wild-type IL2 is set forth in SEQ ID NO: 2;
more preferably, the IL2 mutant has an amino acid sequence that is at least 80% identical to the sequence set forth in any one of SEQ ID NOs: 3 and 8-11.

2. The pharmaceutical composition according to claim 1, wherein,
the linker has an amino acid sequence that is at least 80% identical to the sequence set forth in SEQ ID NO: 12, or the linker has a sequence represented by (G₄S)ₙ, wherein n is selected from 1, 2, 3, 4, 5, or 6;
the antibody Fc block has an N297G mutation, or the antibody Fc block has an amino acid sequence that is at least 80% identical to the sequence set forth in SEQ ID NO: 13;
preferably, the fusion protein has an amino acid sequence that is at least 80% identical to the sequence set forth in any one of SEQ ID NOs: 14-18;
more preferably, the fusion protein forms a homodimer by dimerization of the antibody Fc block.

3. The pharmaceutical composition according to any one of claims 1-2, wherein the concentration of the fusion protein is 1-50 mg/mL.

4. The pharmaceutical composition according to any one of claims 1-3, wherein the buffer is selected from: an acetic acid-sodium acetate buffer, a citric acid-sodium citrate buffer, a histidine-histidine hydrochloride buffer, a succinic acid-sodium succinate buffer, and a disodium hydrogen phosphate-sodium dihydrogen phosphate buffer, preferably an acetic acid-sodium acetate buffer;
the concentration of the buffer is 1-100 mM;
the buffer has a pH value of 4.5-8.0.

5. The pharmaceutical composition according to any one of claims 1-4, wherein the osmotic pressure regulator is selected from: a salt, an amino acid, a sugar or sugar alcohol, or a combination thereof; preferably, the salt is selected from sodium chloride, potassium chloride, or arginine hydrochloride; preferably, the amino acid is selected from glycine, arginine, histidine, glutamic acid, or methionine; preferably, the sugar or sugar alcohol is selected from sucrose, trehalose, mannitol, or sorbitol;
more preferably, the osmotic pressure regulator is selected from sodium chloride, glycine, arginine hydrochloride, sucrose, trehalose, mannitol, or sorbitol, most preferably sucrose or arginine hydrochloride;
the concentration of the sugar or sugar alcohol is 1-15% w/v, and the concentration of the salt is 50-200 mM.

6. The pharmaceutical composition according to any one of claims 1-5, wherein the surfactant is selected from: polysorbate 80 (PS-80), polysorbate 20 (PS-20), or poloxamer; the concentration of the surfactant is 0.01-0.1% w/v.

7. The pharmaceutical composition according to any one of claims 1-6, wherein the pharmaceutical composition comprises: the fusion protein, the acetic acid-sodium acetate buffer, sucrose, and polysorbate-80; preferably, the pharmaceutical composition comprises: 1-50 mg/mL fusion protein, 1-100 mM acetic acid-sodium acetate buffer, 1-15% w/v sucrose, and 0.01-0.1% w/v polysorbate-80, with a pH value of 4.5-6.0.

8. The pharmaceutical composition according to any one of claims 1-6, wherein the pharmaceutical composition comprises: the fusion protein, the acetic acid-sodium acetate buffer, arginine hydrochloride, and polysorbate-80; preferably, the pharmaceutical composition comprises 1-50 mg/mL fusion protein, 1-100 mM acetic acid-sodium acetate buffer, 50-200 mM arginine hydrochloride, and 0.01-0.1% w/v polysorbate-80, with a pH value of 4.5-6.0.

9. The pharmaceutical composition according to any one of claims 1-8, wherein the pharmaceutical composition is an intravenous injection, an intramuscular injection, or a subcutaneous injection, preferably a subcutaneous injection.

10. A method for preparing the pharmaceutical composition according to any one of claims 1-9, wherein the method comprises a step of mixing the fusion protein with the buffer, the osmotic pressure regulator, and the surfactant; preferably, the method comprises a step of exchanging a stock solution of the fusion protein into the buffer by ultrafiltration concentration.

11. A lyophilized formulation, wherein the lyophilized formulation is formed by lyophilizing the pharmaceutical composition according to any one of claims 1-9.

12. A method for preparing the lyophilized formulation according to claim 11, wherein the method comprises a step of lyophilizing the pharmaceutical composition according to any one of claims 1-9.

13. A method for preparing a reconstituted solution of the lyophilized formulation according to claim 11, wherein the method comprises reconstituting the lyophilized formulation according to claim 12 with a solvent; preferably, the solvent is water for injection.

14. A reconstituted solution of an Fc fusion protein comprising an IL2 mutant prepared by the method according to claim 13.

15. Use of the pharmaceutical composition according to any one of claims 1-9, the lyophilized formulation according to claim 11, or the reconstituted solution according to claim 14 in the manufacture of a medicament for treating an autoimmune disease or a proliferative disease, wherein preferably, the autoimmune disease is selected from rheumatoid arthritis, ankylosing spondylitis, systemic lupus erythematosus, cutaneous lupus erythematosus, lupus nephritis, IgA nephropathy, Sjogren's syndrome, polymyositis, dermatomyositis, scleroderma, psoriasis, plaque psoriasis, alopecia areata, multiple sclerosis, amyotrophic lateral sclerosis, inflammatory bowel disease, ulcerative colitis, Crohn's disease, graft-versus-host disease, organ transplant rejection, autoimmune hepatitis, type I diabetes, autoimmune vasculitis, eczema, or asthma;
preferably, the proliferative disease is selected from a neoplasm, a solid tumor, a hematologic tumor, malignant ascites, or malignant pleural effusion; wherein the solid tumor can be benign or malignant, primary or metastatic, and the malignant solid tumor can be a cancer or a sarcoma, such as epithelial cell carcinoma, endothelial cell carcinoma, squamous cell carcinoma, teratoma, lung tumor, papillomavirus-induced cancer, adenocarcinoma, carcinoma, melanoma, angiosarcoma, neuroblastoma, metastatic lung cancer, non-small cell lung cancer, small cell lung cancer, breast cancer, Merkel cell carcinoma, ovarian cancer, renal cell carcinoma, metastatic renal cancer, head and neck cancer, bladder cancer, or non-muscle invasive bladder cancer; the hematologic tumor can be selected from leukemia, lymphoma, and multiple myeloma, such as B-cell lymphoma, T-cell lymphoma, cutaneous T-cell lymphoma, and T-cell large granular lymphocytic leukemia.

16. The pharmaceutical composition according to any one of claims 1-9, the lyophilized formulation according to claim 11, or the reconstituted solution according to claim 14 for use in treating an autoimmune disease or a proliferative disease, wherein
preferably, the autoimmune disease is selected from rheumatoid arthritis, ankylosing spondylitis, systemic lupus erythematosus, cutaneous lupus erythematosus, lupus nephritis, IgA nephropathy, Sjogren's syndrome, polymyositis, dermatomyositis, scleroderma, psoriasis, plaque psoriasis, alopecia areata, multiple sclerosis, amyotrophic lateral sclerosis, inflammatory bowel disease, ulcerative colitis, Crohn's disease, graft-versus-host disease, organ transplant rejection, autoimmune hepatitis, type I diabetes, autoimmune vasculitis, eczema, or asthma;
preferably, the proliferative disease is selected from a neoplasm, a solid tumor, a hematologic tumor, malignant ascites, or malignant pleural effusion; wherein the solid tumor can be benign or malignant, primary or metastatic, and the malignant solid tumor can be a cancer or a sarcoma, such as epithelial cell carcinoma, endothelial cell carcinoma, squamous cell carcinoma, teratoma, lung tumor, papillomavirus-induced cancer, adenocarcinoma, carcinoma, melanoma, angiosarcoma, neuroblastoma, metastatic lung cancer, non-small cell lung cancer, small cell lung cancer, breast cancer, Merkel cell carcinoma, ovarian cancer, renal cell carcinoma, metastatic renal cancer, head and neck cancer, bladder cancer, or non-muscle invasive bladder cancer; the hematologic tumor can be selected from leukemia, lymphoma, and multiple myeloma, such as B-cell lymphoma, T-cell lymphoma, cutaneous T-cell lymphoma, and T-cell large granular lymphocytic leukemia.

17. A method for treating an autoimmune disease, wherein the method comprises administering to a subject an effective amount of the pharmaceutical composition according to any one of claims 1-9 or the reconstituted solution according to claim 14; preferably, the autoimmune disease is selected from rheumatoid arthritis, ankylosing spondylitis, systemic lupus erythematosus, cutaneous lupus erythematosus, lupus nephritis, IgA nephropathy, Sjogren's syndrome, polymyositis, dermatomyositis, scleroderma, psoriasis, plaque psoriasis, alopecia areata, multiple sclerosis, amyotrophic lateral sclerosis, inflammatory bowel disease, ulcerative colitis, Crohn's disease, graft-versus-host disease, organ transplant rejection, autoimmune hepatitis, type I diabetes, autoimmune vasculitis, eczema, or asthma; preferably, the effective amount is 0.001-10 mpk.

18. A method for treating a proliferative disease, wherein the method comprises administering to a subject an effective amount of the pharmaceutical composition according to any one of claims 1-9 or the reconstituted solution according to claim 14; preferably, the proliferative disease is selected from a neoplasm, a solid tumor, a hematologic tumor, malignant ascites, or malignant pleural effusion; wherein the solid tumor can be benign or malignant, primary or metastatic, and the malignant solid tumor can be a cancer or a sarcoma, such as epithelial cell carcinoma, endothelial cell carcinoma, squamous cell carcinoma, teratoma, lung tumor, papillomavirus-induced cancer, adenocarcinoma, carcinoma, melanoma, angiosarcoma, neuroblastoma, metastatic lung cancer, non-small cell lung cancer, small cell lung cancer, breast cancer, Merkel cell carcinoma, ovarian cancer, renal cell carcinoma, metastatic renal cancer, head and neck cancer, bladder cancer, or non-muscle invasive bladder cancer; the hematologic tumor can be selected from leukemia, lymphoma, and multiple myeloma, such as B-cell lymphoma, T-cell lymphoma, cutaneous T-cell lymphoma, and T-cell large granular lymphocytic leukemia.

19. An article of manufacture, comprising a container, wherein the container comprises the pharmaceutical composition according to any one of claims 1-9, the lyophilized formulation according to claim 11, or the reconstituted solution according to claim 14.
